# FASCICULE DE BREVET EUROPEEN

(11) **EP 3 313 459 B1**
(45) Date de publication et mention de la délivrance du brevet: **01.05.2019**
(21) Numéro de dépôt: 16745779.5
(22) Date de dépôt: 23.06.2016
(51) Int. Cl.: A61L 15/26, A61L 15/58, A61L 15/28

(54) **ARTICLE ADHÉSIF SUR LA PEAU**
AUF HAUT HAFTENDER ARTIKEL
SKIN-ADHESIVE ITEM

(30) Priorité: 26.06.2015 FR 1501350
(43) Date de publication de la demande: 02.05.2018
(73) Titulaire: ELKEM SILICONES France SAS, 69003 Lyon (FR)
(72) Inventeur: MOINE, Caroline, 42290 Sorbiers (FR); QUEMIN, Maryline, 69100 Villeurbanne (FR)
(74) Mandataire: Mekki, Boualem
(86) Numéro de dépôt international: PCT/FR2016/000105
(87) Numéro de publication internationale: WO 2016/207498

(56) Documents cités:
- WO-A1-2011/092404
- FR-A1- 2 899 248
- "Soft Skin Adhesive Gel for Scar Care and Wound Management HC2 2022 A&B Technical Data Sheet n° SIL 15 037 3 -March 2015", , mars 2015 (2015-03), XP055268817, Extrait de l'Internet: URL:http://www.silbione.com/wp-content/upl oads/2014/01/Silbione_HC2_2022_AB_SIL15037 3.pdf [extrait le 2016-04-26]

## Description

Le domaine de l'invention est de celui des articles adhésifs à la peau pour un usage médical ou paramédical.

L'utilisation des gels silicone est très importante dans le domaine médical. En effet les propriétés intrinsèques des gels silicone font qu'ils adhèrent rapidement à une peau sèche, mais ne collent pas sur la surface d'une plaie humide, ne provoquant pas ainsi de dommage lors de leurs retraits. Les gels silicones présentent aussi l'avantage de pouvoir être assemblés à un grand nombre de supports tout en étant inertes pour l'organisme, évitant ainsi tout problème de toxicité lorsqu'ils sont utilisés chez l'être humain. Les gels silicone sont entre autres utilisés pour le traitement des plaies ou cicatrices car ils apportent au dispositif médical des propriétés facilitant la guérison du patient en maintenant un milieu humide autour de la plaie et permettent ainsi de maintenir l'hydratation des tissus lésés.

Pour ces applications le substrat doit être biocompatible, flexible et avoir de bonnes propriétés mécaniques. Les films en polyuréthane sont très utilisés car ils répondent à l'ensemble de ces exigences techniques notamment :
- une bonne biocompatibilité,
- une bonne perméabilité à la vapeur d'eau (gestion des fluides entre le pansement et le milieu extérieur pour éviter au pansement de gonfler ou de se décoller),
- de bonnes propriétés mécaniques (résistance à la traction, capacité d'élongation),
- un toucher doux, et
- une bonne flexibilité.

La mise en oeuvre de ces produits passe par une étape d'enduction sur un substrat. L'enduction des gels silicones sur ces supports en polyuréthane, est une étape clé du procédé de fabrication du dispositif médical et les propriétés de ce composite doivent être bonnes. En effet, le gel doit être suffisamment adhésif à la peau pour maintenir le dispositif en place. Il doit aussi suffisamment adhérer au substrat pour éviter la génération de résidus au retrait du dispositif.

Cependant, il est connu que l'adhésion des gels silicones sur des supports en plastique ayant une faible énergie de surface est difficile à obtenir. Or l'adhésion au substrat est fondamentale pour assurer la bonne cohésion de l'article adhésif à la peau, notamment pour un usage médical ou paramédical, et éviter la présence de résidus au retrait de l'article.

Ainsi, lorsque des gels silicones sont utilisés comme éléments d'enduction de supports en polyuréthane ou comme couche apposée sur ces supports, il est souhaitable de renforcer leur adhérence aux supports en polyuréthane, de manière à ce que les produits ainsi traités résistent efficacement aux contraintes physiques.

Il est par exemple connu que l'adhésion des gels silicone sur un substrat plastique est améliorée en appliquant sur la surface du support un traitement Corona afin de modifier l'énergie de surface dudit support. Cette technique consiste à oxyder la surface du matériau afin d'améliorer la mouillabilité en augmentant la tension de surface. Cependant, le niveau d'adhérence obtenu n'est pas toujours suffisant pour certaines applications.

Une autre voie pour améliorer l'adhérence des gels silicones sur des supports en plastique, par exemple en polyuréthane, consiste à utiliser un primaire d'adhérence, connus aussi sous le nom de « primaire d'accrochage ». Cette approche technique permet d'obtenir des produits offrant un niveau d'accroche légèrement amélioré par rapport à un substrat polymérique soumis à un traitement Corona. Parmi les primaires existants à la date on peut citer :
- Les primaires formulés en milieu solvant. Un exemple est décrit dans la demande de brevet WO 2011/092404 de la société Bluestar Silicones France où un primaire est constitué d'une matière active (huile organopolysiloxane à fonction hydrogénosilylé (SiH) et Si-alcényle ou une résine silicone ayant des fonctions hydrogénosilylé) diluée dans un solvant silicone (cyclopentasiloxane). Ce primaire est très performant mais doit être utilisé dans des conditions bien précises (dilution de matière active, poids de primaire enduit) pour qu'un bon compromis de propriétés (adhésion sur le substrat et conservation du tack) soit atteint. De plus, un autre inconvénient de ce type de primaire est sa teneur en solvant qui rend son utilisation plus difficile lors de l'étape d'enduction ; et
- les primaires en élastomère de silicone qui sont préparés à partir de compositions précurseurs réticulant par une réaction d'hydrosilylation comprenant des promoteurs d'adhésion qui sont le plus souvent des silanes qui permettent d'améliorer l'adhésion sur divers substrats (en polyamide, en polyester ou en polyuréthane). Cependant, lors de la préparation de l'élastomère silicone la condensation des silanes libère des produits secondaires (alcools) qui rend l'utilisation de ce type de primaire plus difficile lors de l'étape d'enduction.

Selon une autre approche technique, par exemple décrite dans la demande de brevet WO 2005/051442 de la société Dow Corning Corp., l'adhérence d'un gel silicone à la surface d'un substrat polymérique plastique tels que des films en polyuréthane est améliorée en traitant directement le substrat ou le gel silicone par une mise en contact avec des dérivés: titanate, zirconate, siloxanes ayant des fonctions hydrogénosylilées ou du platine tel qu'un complexe platine-(0)-1,3-diéthenyl-1,1,3,3-tetraméthyldisiloxane. Il est à remarquer que l'utilisation de primaires d'adhérence de type titanate, tel que le tétrabutanoate de titane, est très répandue mais pose des problèmes lors de leur mise en oeuvre.

Une solution au problème énoncé ci-dessus est exposé dans la demande de brevet WO 2014/131999 de la société Urgo qui décrit un article comprenant au moins un substrat polymérique assemblé à au moins une couche de polymère de silicone, caractérisé en ce que l'un au moins du substrat polymérique ou de la couche de polymère de silicone a été mis en contact avec des particules de dioxyde de titane, d'oxyde de magnésium et/ou d'oxyde de zinc, avant assemblage dudit article, et en ce que l'un au moins du substrat polymérique ou de la couche de polymère de silicone a été mis en contact avec de l'eau avant ou après assemblage dudit article. Les résultats d'adhésion présentent une nette amélioration comparée à un traitement Corona ou à l'imprégnation d'un support non tissé à base de polyéthylène imprégné avec du tétrabutanoate de titane à 5% en poids dans l'isopropanol.

Il est également possible d'utiliser d'autres types de primaires d'adhérence formulés en phase aqueuse mais les résultats en termes d'amélioration d'adhérence ne sont pas satisfaisants, et nécessite en outre l'incorporation d'additifs (comme par exemple de l'acide acétique) lors de leur mise en oeuvre et/ou l'application d'un traitement thermique, traitement incompatible avec l'utilisation de certains substrats polymériques.

Ainsi, l'utilisation de primaires d'adhérence de différents types et notamment de type titanate, engendre bon nombre de problèmes ou de complications liés à leur mise en oeuvre et à leur utilisation. En outre, les niveaux d'adhérence obtenus par ces traitements nécessitent encore d'être améliorés. La Demanderesse a ainsi cherché à mettre au point un nouveau primaire d'adhérence offrant une adhésion améliorée entre un gel de silicone et un film souple en polyuréthane n'utilisant pas de promoteurs d'adhésion de type titanate ou silanes ni même de solvant et offrant les meilleures garanties possibles en matière de sécurité sanitaire.

Ainsi, la présente invention a pour objet de proposer de nouveaux articles adhésifs à la peau comprenant un support en polyuréthane enduit d'un primaire spécifique et une couche de polymère de silicone se présentant sous la forme de gel de silicone, dans lesquels l'adhérence du silicone sur le support en polyuréthane est particulièrement élevée, lesdits substrats convenant par ailleurs aux applications médicales, paramédicales ou cosmétiques sans générer de problèmes de toxicité liés à l'emploi de certains solvants, silanes ou titanates.

Un autre objectif de la présente invention est de proposer de nouveaux articles adhésifs à la peau qui peuvent être utilisés en tant que couche de contact avec la peau dans différents types de pansements ou de dispositifs médicaux.

Un autre objectif de la présente invention consiste à fournir de nouveaux articles adhésifs à la peau dans lesquels les propriétés de tack du gel silicone sont suffisantes pour permettre une bonne adhésion à la peau.

Un autre objectif de la de la présente invention est de fournir un procédé de préparation dudit article adhésif sur la peau.

Ces objectifs sont atteints par l'invention qui concerne un article adhésif sur la peau comprenant :
- un support **S** qui est un film souple en polyuréthane ayant une face de dessus **S1** et une face de dessous **S2,**
- au moins un primaire d'accrochage **C1** appliqué sur au moins une partie ou sur la totalité de la face de dessus **S1** dudit support **S**,
- au moins une couche **D1** constituée par un gel silicone **E** obtenu par réticulation d'une composition silicone **Y** réticulable par une réaction d'hydrosilylation qui a été appliquée sur ledit primaire d'accrochage **C1** à l'opposé dudit support **S**, et
- éventuellement au moins une couche de protection F constituée d'un matériau de protection pelable et appliquée sur ladite couche **D1,**
- ledit primaire d'accrochage **C1** étant caractérisé en ce qu'il est constitué d'un élastomère silicone qui n'est pas un gel silicone et qui est obtenu par réticulation d'une composition silicone **X** constituée de:
   1) au moins un organopolysiloxane **A** comportant, par molécule, au moins deux radicaux vinyles liés chacun à un atome de silicium et constitué :
      (i) d'au moins deux motifs siloxy de formule (A1) :

         YₐZ_{b}SiO_{(4-(a+b)/2} (A1)

         dans laquelle :
         - Y représente un groupe alcényle en C₂ à C₆, et de préférence un groupe vinyle,
         - Z représente un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle,
         - a et b représentent des nombres entiers, a valant 1, 2 ou 3, b valant 0, 1 ou 2 et (a+b) valant 1, 2 ou 3 ;
      (ii) et comportant éventuellement d'autres motifs siloxy de formule (A2) :

         Z_{c}SiO_{(4-c)/2} (A2)

         dans laquelle :
         - Z a la même signification que ci-dessus, et
         - c représente un nombre entier valant 0, 1, 2 ou 3.
   2) au moins un organopolysiloxane **B** comportant, par molécule, au moins trois atomes d'hydrogène liés chacun à un atome de silicium et constitué :
      (i)d'au trois motifs siloxy de formule (B1) :

         HZ_{b}SiO_{(3-b)/2} (B1)

         dans laquelle :
         - H est un atome d'hydrogène,
         - Z représente un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle,
         - b valant 0, 1 ou 2;
      (ii) et comportant éventuellement d'autres motifs siloxy de formule (B2) :

         Z_{c}SiO_{(4-c)/2} (B2)

         dans laquelle :
         - Z a la même signification que ci-dessus, et
            c représente un nombre entier valant 0, 1, 2 ou 3.
   3) au moins un catalyseur **C** d'hydrosilylation,
   4) au moins un inhibiteur **D** de la réaction d'hydrosilylation,
   5) éventuellement au moins un additif stabilisant **K,** et
   7) éventuellement au moins une résine silicone **M** ayant des groupements hydroxyles et exempt de groupement alcényles ou hydrogénosilyles SiH, et
      - avec la condition que les quantités en poids des organopolysiloxanes **A** et **B** sont déterminées de manière à ce que le rapport RHalk = nH/tAlk et 1,00 ≤ RHalk ≤ 4,5 avec :
         - nH = nombre de mole d'atome d'hydrogène directement lié à un atome de silicium de l'organopolysiloxane **B,** et
         - tAlk = nombre de mole d'alcényle directement lié à un atome de silicium de l'organopolysiloxane **A.**

La Demanderesse a mis en oeuvre d'importants moyens de recherche et de nombreuses expérimentations pour atteindre cet objectif parmi d'autres. Et au terme de cela, elle a eu le mérite de trouver, de manière tout à fait surprenante et inattendue, qu'en utilisant sur un film souple en plastique de polyuréthane de faible énergie de surface un primaire d'accrochage selon l'invention, permet d'obtenir une adhésion satisfaisante lorsqu'un gel silicone est ensuite enduit sur ledit primaire.

Ceci est d'autant plus surprenant que la composition silicone **X** selon l'invention est exempte de titanate, de silicone bi fonctionnel ou de silane présentés par l'art antérieur comme des composants essentiels pour ce type d'adhésion recherché.

L'absence de titanate ou de silicone bi fonctionnel, tels que ceux présentés dans la demande de brevet WO 2011/092404, dans la composition silicone **X** selon l'invention permet de s'affranchir d'une utilisation de solvant organique évitant ainsi bon nombre de problèmes d'intolérance voire de toxicité liés à l'emploi des solvants organiques dans la fabrication d'article à usage médical. Cet aspect fait l'objet d'une attention toute particulière par l'industrie du pansement à usage médical ou paramédical.

L'absence de silane dans la composition silicone **X** permet d'éviter la libération d'alcool due à la condensation des silanes et ce qui est un avantage lors de la fabrication des supports enduits.

Un autre avantage lié à l'utilisation comme primaire d'accrochage d'un élastomère silicone obtenu par réticulation d'une composition silicone **X** selon l'invention est que le tack du gel silicone enduit sur le primaire selon l'invention n'est pas dégradé. Le terme « tack », ou "collant instantané", détermine le pouvoir adhésif à des temps courts. Pour apprécier et évaluer le tack, les méthodes dites de « Probe Tack » ont été développées. Le principe de ces tests consiste à mettre en contact la surface d'un poinçon métallique rigide avec le gel silicone et à enregistrer l'évolution de la force au cours de l'étape de séparation. L'intégration de la courbe représentant la force en fonction du déplacement du poinçon conduit à l'énergie de tack (unité en mJ/cm²).

Au sens de la présente invention, l'expression « gel silicone » désigne un produit silicone réticulé ne présentant aucun écoulement lorsqu'il est à l'état stable et est caractérisé par un taux de pénétration (ou « pénétrabilité ») compris entre 80 et 300 dixièmes de mm. Elle se mesure par pénétrométrie selon la norme NF ISO 2137, à l'aide d'un pénétromètre modèle PNR 12 Petrotest avec un poids total de la tige et du cône fixé à 62,5 g. La pénétrabilité au cône d'un gel silicone est déterminée à 25 °C en mesurant la profondeur de pénétration du cône dans l'échantillon, celle-ci étant obtenue en libérant l'ensemble cône du pénétromètre et en laissant le cône agir pendant 5 secondes.

De préférence, les quantités de la composition silicone **X** appliquées sur le support S seront déterminées de manière à obtenir des enduction ayant une teneur en composition silicone **X** avant réticulation comprise entre 1 et 100 g/m² de support, de préférence entre 1 et 50 g/m² et encore plus préférentiellement entre 2 et 30 g/m².

Selon un mode de réalisation avantageux, les quantités en poids des organopolysiloxanes **A** et **B** sont déterminées de manière à ce que 1,50 ≤ RHalk ≤ 4,0. De préférence, les quantités en poids des organopolysiloxanes **A** et **B** sont déterminées de manière à ce que 1,50 ≤ RHalk ≤ 2,5.

L'organopolysiloxane **A** peut présenter une structure linéaire, ramifié, cycliques ou en réseau. Lorsqu'il s'agit de polyorganosiloxanes linéaires, ceux-ci peuvent être essentiellement constitués :
- de motifs siloxyles « D » choisi parmi les motifs de formules R₂SiO_{2/2}, RZSiO_{2/2} et Z₂SiO_{2/2};
- de motifs siloxyles « M » choisis parmi les motifs de formules R₃SiO_{1/2}, R₂ZSiO_{1/2}, RZ₂SiO_{1/2} et R₃SiO_{2/2},
- le symbole Z représente un groupe alcényle en C₂ à C₆, et de préférence un groupe vinyle et le symbole R un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle.

A titre d'exemples de motifs « D », on peut citer les groupes diméthylsiloxy, méthylphénylsiloxy, méthylvinylsiloxy, méthylbuténylsiloxy, méthylhexénylsiloxy, méthyldécénylsiloxy et méthyldécadiénylsiloxy.

A titre d'exemple de motifs « M », on peut citer les groupes triméthylsiloxy, diméthylphénylsiloxy, diméthylvinylsiloxy et diméthylhexénylsiloxy.

Ces organopolysiloxanes, en particulier lorsqu'ils sont linéaires, peuvent être des huiles ayant une viscosité dynamique à 25°C comprise entre 1 mPa.s et 100 000 mPa.s, préférentiellement entre 10 mPa.s et 10 000 mPa.s et encore plus préférentiellement entre 50 mPa.s et 5000 mPa.s.

Toutes les viscosités dont il est question dans le présent exposé correspondent à une grandeur de viscosité dynamique à 25°C dite "Newtonienne", c'est-à-dire la viscosité dynamique qui est mesurée, de manière connue en soi, avec un viscosimètre Brookfield à un gradient de vitesse de cisaillement suffisamment faible pour que la viscosité mesurée soit indépendante du gradient de vitesse.

Lorsqu'il s'agit d'un organopolysiloxane cyclique, celui-ci peut être constitué de motifs siloxyles « D » choisi parmi les motifs de formules R₂SiO_{2/2}, RZSiO_{2/2} et Z₂SiO_{2/2}. R et Z ont les mêmes significations que ci-dessus. Des exemples de tels motifs « D » sont décrits ci-dessus. Ce polyorganosiloxane cyclique peut avoir une viscosité dynamique à 25°C comprise entre 1 mPa.s et 5 000 mPa.s.

Des exemples d'organopolysiloxane **A** sont :
- les polydiméthylsiloxanes à extrémités diméthylvinylsilyles ;
- les poly(méthylphénylsiloxane-co-diméthylsiloxane) à extrémités diméthylvinylsilyles ;
- les poly(vinylméthylsiloxane-co-diméthylsiloxane) à extrémités diméthylvinylsilyles ;
- les poly(diméthylsiloxane-co-vinylméthylsiloxane) à extrémités triméthylsilyles ;
- les polyméthylvinylsiloxanes cycliques.

L'organopolysiloxane **B** comportant, par molécule, au moins trois atomes d'hydrogène liés chacun à un atome de silicium et constitué :
(i)d'au moins trois motifs siloxy de formule (B1) :

   HZ_{b}SiO_{(3-b)/2} (B1)

   dans laquelle :
   - H est un atome d'hydrogène,
   - Z représente un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle,
   - b valant 0, 1 ou 2;
(ii) et comportant éventuellement d'autres motifs siloxy de formule (B2) :

   Z_{c}SiO_{(4-c)/2} (B2)

   dans laquelle :
   - Z a la même signification que ci-dessus, et c représente un nombre entier valant 0, 1, 2 ou 3.

Des exemples de motifs de formule (B1) sont les suivants : H(CH₃)₂SiO_{1/2}, HCH₃SiO_{2/2} et HSiO_{3/2}.

L'organopolysiloxane **B** peut présenter une structure linéaire, ramifiée, cyclique ou en réseau. Lorsqu'il s'agit de polyorganosiloxanes linéaires, ceux-ci peuvent être essentiellement constitués :
- de motifs siloxyles « D » choisi parmi les motifs de formules HZSiO_{2/2} et Z₂SiO_{2/2} ;
- de motifs siloxyles « M » choisis parmi les motifs de formules HZ₂SiO_{1/2} et Z₃SiO_{1/2},
- avec le symbole Z ayant la même signification que ci-dessus et le symbole H désignant un atome d'hydrogène.

Ces polyorganosiloxanes linéaires peuvent être des huiles ayant une viscosité dynamique à 25°C comprise entre 1 mPa.s et 100 000 mPa.s, préférentiellement entre 1 mPa.s et 5 000 mPa.s, et encore plus préférentiellement entre 1 mPa.s et 2000 mPa.s.

Lorsqu'il s'agit de polyorganosiloxanes cycliques, ceux-ci peuvent être constitués de motifs siloxyles « D » choisi parmi les motifs de formules Z₂SiO_{2/2} et de motifs siloxyle de formule HZSiO_{2/2} uniquement. Z a la même signification que ci-dessus. Ces polyorganosiloxanes cycliques peuvent avoir une viscosité dynamique à 25°C comprise entre 1 mPa.s et 5 000 mPa.s.

Des exemples d'organopolysiloxane **B** sont :
- les polydiméthylsiloxanes à extrémités hydrogénodiméthylsilyles ;
- les poly(diméthylsiloxane-co-hydrogénométhylsiloxane) à extrémités triméthylsilyles ;
- les poly(diméthylsiloxane-co-hydrogénométhylsiloxane) à extrémités hydrogénodiméthyl-silyles ;
- les polyhydrogénométhylsiloxanes à extrémités triméthylsilyles ;
- les hydrogénométhylpolysiloxanes cycliques.

Lorsqu'il s'agit de polyorganosiloxanes ramifiés ou en réseaux, ceux-ci peuvent comprendre en outre :
- des motifs siloxyles « T » choisis parmi les motifs de formules HSiO_{3/2} et ZSiO_{3/2} ;
- des motifs siloxyles « Q » de formule SiO_{4/2},
- avec le symbole H représentant un atome d'hydrogène et Z ayant la même signification que ci-dessus.

Comme catalyseur C d'hydrosilylation utile selon l'invention, on peut citer les composés d'un métal appartenant au groupe du platine bien connu de l'homme de l'art. Les métaux du groupe du platine sont ceux connus sous le nom de platinoïdes, appellation qui regroupe, outre le platine, le ruthénium, le rhodium, le palladium, l'osmium et l'iridium. On utilise, de préférence, les composés du platine et du rhodium. On peut, en particulier, utiliser les complexes du platine et d'un produit organique décrit dans les brevets US-A-3 159 601, US-A-3 159 602, US-A-3 220 972 et les brevets européens EP-A-0 057 459, EP-A-0 188 978 et EP-A-0 190 530, les complexes du platine et d'organosiloxanes vinylés décrits dans les brevets US-A-3 419 593. Le catalyseur généralement préféré est le platine. A titre d'exemples, on peut citer le platine noir, l'acide chloroplatinique, un acide chloroplatinique modifié par un alcool, un complexe de l'acide chloroplatinique avec une oléfine, un aldéhyde, un vinylsiloxane ou un alcool acétylénique, entre autres. La préférence va à la solution ou complexe de Karstedt, tel que décrit dans le brevet US-A-3 775 452, à l'acide chloroplatinique hexahydrate ou un catalyseur au platine comprenant des ligands carbènes.

Comme inhibiteur **D** de la réaction d'hydrosilylation utile selon l'invention, on peut citer celui choisi parmi les alcools α-acétyléniques, les diesters α-α'-acétyléniques, les composés conjugués ène-yne, les cétones α-acétyléniques, les acrylonitriles, les maléates, les fumarates et les mélanges de ceux-ci. Ces composés capables de remplir la fonction d'inhibiteur d'hydrosilylation sont bien connus de l'homme du métier. Ils peuvent être utilisés seuls ou en mélanges.

Un inhibiteur **D** de type alcool α-acétylénique peut être choisi parmi les composés de formule (D1) suivante :

(R¹)(R²)C(OH)-C≡CH (D1)

dans laquelle :
- le groupe R¹ représente un groupe alkyle, un groupe cycloalkyle, un groupe (cycloalkyl)alkyle, un groupe aryle ou un groupe arylalkyle,
- le groupe R² représente un atome d'hydrogène, un groupe alkyle, un groupe cycloalkyle, un groupe (cycloalkyl)alkyle, un groupe aryle ou un groupe arylalkyle,
- ou bien R¹ et R² constituent ensemble avec l'atome de carbone auquel ils sont liés un cycle aliphatique à 5, 6, 7 ou 8 chaînons, éventuellement substitué une ou plusieurs fois.

Selon la formule (D1) :
- Par « alkyle », on entend une chaîne hydrocarbonée saturée contenant de 1 à 20 atomes de carbone, de préférence de 1 à 8 atomes de carbone. Un groupe alkyle peut être choisi dans le groupe constitué par méthyle, éthyle, isopropyle, n-propyle, tert-butyle, isobutyle, n-butyle, n-pentyle, isoamyle et 1,1-diméthylpropyle ;
- Par « cycloalkyle », on entend selon l'invention un groupe hydrocarboné saturé monocyclique ou polycyclique, de préférence monocyclique ou bi cyclique, contenant de 3 à 20 atomes de carbone, de préférence de 5 à 8 atomes de carbone. Lorsque le groupe cycloalkyle est polycyclique, les multiples noyaux cycliques peuvent être rattachés les uns aux autres par une liaison covalente et/ou par un atome spinanique et/ou être condensés les uns aux autres. Un groupe cycloalkyle peut être choisi dans le groupe constitué par le cyclopropyle, le cyclobutyle, le cyclopentyle, le cyclohexyle, le cycloheptyle, le cyclooctyle, l'adamantane et le norborane ;
- Par « (cycloalkyl)alkyle », on entend selon l'invention un groupe cycloalkyle tel que défini ci-avant lié à un groupe alkyle tel que défini ci-avant également.
- Par « aryle », on entend selon l'invention un groupe hydrocarboné aromatique contenant de 5 à 18 atomes de carbone, monocyclique ou polycyclique. Un groupe aryle peut être choisi dans le groupe constitué par phényle, naphtyle, anthracényle et phénanthryle ;
- Par « arylalkyle », on entend selon l'invention un groupe aryle tel que défini ci-avant lié à un groupe alkyle tel que défini ci-avant également.

Selon un mode de réalisation préféré, dans la formule (D1) R¹ et R² constituent ensemble avec l'atome de carbone auquel ils sont liés un cycle aliphatique non substitué à 5, 6, 7 ou 8 chaînons. Selon un autre mode de réalisation préféré, R¹ et R², identiques ou différents, représentent indépendamment l'un de l'autre un groupe alkyle monovalent en C₁ à C₁₂, de préférence en C₁ à C₆.

Un inhibiteur **D** qui est un alcool α-acétylénique utile selon l'invention peut être choisi dans le groupe constitué par les composés suivants : 1-éthynyl-1-cyclopentanol ; 1-éthynyl-1-cyclohexanol (aussi appelé ECH) ; 1-éthynyl-1-cycloheptanol ; 1-éthynyl-1-cyclooctanol ; 3-méthyl-1-butyn-3-ol (aussi appelé MBT) ; 3-méthyl-1-pentyn-3-ol ; 3-méthyl-1-hexyn-3-ol ; 3-méthyl-1-heptyn-3-ol ; 3-méthyl-1-octyn-3-ol ; 3-méthyl-1-nonyn-3-ol ; 3-méthyl-1-decyn-3-ol ; 3-méthyl-1-dodecyn-3-ol ; 3-méthyl-1-pentadecyn-3-ol ; 3-éthyl-1-pentyn-3-ol ; 3-éthyl-1-hexyn-3-ol ; 3-éthyl-1-heptyn-3-ol ; 3,5-diméthyl-1-hexyn-3-ol ; 3-isobutyl-5-méthyl-1-hexyn-3-ol ; 3,4,4-triméthyl-1-pentyn-3-ol ; 3-éthyl-5-méthyl-1-heptyn-3-ol ; 3,6-diéthyl-1-nonyn-3-ol ; 3,7,11-triméthyl-1-dodecyn-3-ol (aussi appelé TMDDO) ; 1,1-diphényl-2-propyn-1-ol ; 3-butyn-2-ol ; 1-pentyn-3-ol ; 1-hexyn-3-ol ; 1-heptyn-3-ol ; 5-méthyl-1-hexyn-3-ol ; 4-éthyl-1-octyn-3-ol et 9-éthynyl-9-fluorenol.

Un inhibiteur **D** de type diester α-α'-acétylénique peut être choisi parmi les composés de formule (D2) suivante : 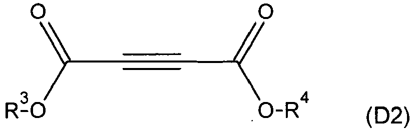 dans laquelle les groupe R³ et R⁴, identiques ou différents, représentent indépendamment l'un de l'autre un groupe alkyle, un groupe cycloalkyle, un groupe (cycloalkyl)alkyle, un groupe aryle, un groupe arylalkyle ou un groupe silyle.

Par « silyle », on entend selon l'invention un groupe de formule -SiR₃, chaque R représentant indépendamment un groupe alkyle contenant de 1 à 20 atomes de carbone, de préférence de 1 à 8 atomes de carbone. Un groupe silyle peut être par exemple le groupe triméthylsilyle.

Selon un mode de réalisation particulier, dans la formule (D2) R³ et R⁴, identiques ou différents, représentent indépendamment l'un de l'autre un groupe alkyle en C₁ à C₁₂, de préférence en C₁ à C₆, ou le groupe triméthylsilyle. Un inhibiteur **D** qui est un diester α-α'-acétylénique utile selon l'invention peut être choisi dans le groupe constitué par les composés suivants : l'acétylène-dicarboxylate de diméthyle (DMAD), l'acétylène-dicarboxylate de diéthyle, l'acétylène-dicarboxylate de tert-butyle et l'acétylène-dicarboxylate de bis(triméthylsilyle).

Un inhibiteur **D** de type composé conjugué ène-yne peut être choisi parmi les composés de formule (D3) suivante : 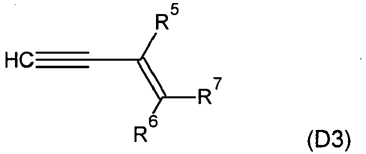 dans laquelle :
- les groupes R⁵, R⁶ et R⁷ représentent, indépendamment les uns des autres, un atome d'hydrogène, un groupe alkyle, un groupe cycloalkyle, un groupe (cycloalkyl)alkyle, un groupe aryle ou un groupe arylalkyle,
- ou bien au moins deux groupes parmi les groupes R⁵, R⁶ et R⁷ constituent ensemble avec le ou les atomes de carbone auxquels ils sont liés un cycle aliphatique à 5, 6, 7 ou 8 chaînons, éventuellement substitué une ou plusieurs fois.

Selon un mode de réalisation particulier, les groupes R⁵, R⁶ et R⁷ représentent, indépendamment les uns des autres, un atome d'hydrogène, un groupe alkyle en C₁ à C₁₂, de préférence en C₁ à C₆, ou un groupe aryle. Un inhibiteur **D** qui est un composé conjugué ène-yne utile selon l'invention peut être choisi dans le groupe constitué par les composés suivants : le 3-méthyl-3-pentène-1-yne ; le 3-méthyl-3-hexène-1-yne ; le 2,5-diméthyl-3-hexène-1-yne ; le 3-éthyl-3-butène-1-yne ; et le 3-phényl-3-butène-1-yne. Selon un autre mode de réalisation particulier, deux groupes choisis parmi les groupes R⁵, R⁶ et R⁷ constituent ensemble avec le ou les atomes de carbone auxquels ils sont liés un cycle aliphatique non substitué à 5, 6, 7 ou 8 chaînons et le troisième groupe restant représente un atome d'hydrogène ou un groupe alkyle en C₁ à C₁₂, de préférence en C₁ à C₆. Un inhibiteur **D** qui est un composé conjugué ène-yne utile selon l'invention peut être le 1-éthynyl-1-cyclohexène.

Un inhibiteur **D** de type cétone α-acétylénique peut être choisi parmi les composés de formule (D4) suivante : 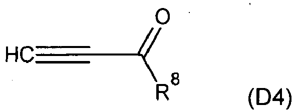 dans laquelle R⁸ représente un groupe alkyle, un groupe cycloalkyle, un groupe (cycloalkyl)alkyle, un groupe aryle ou un groupe arylalkyle, les groupes alkyles, cycloalkyles, (cycloalkyl)alkyle, aryle ou arylalkyle pouvant éventuellement être substitué une ou plusieurs fois par un atome de chlore, de brome ou d'iode.
Selon un mode de réalisation préféré, R⁸ représente un groupe alkyle monovalent en C₁ à C₁₂, de préférence en C₁ à C₆, éventuellement être substitué une ou plusieurs fois par un atome de chlore ou de brome, ou un groupe cycloalkyle, ou un groupe aryle. Un inhibiteur **D** qui est une cétone α-acétylénique utile selon l'invention peut être choisi dans le groupe constitué par les composés suivants : la 1-octyn-3-one, la 8-chloro-1-octyn-3-one ; la 8-bromo-1-octyn-3-one ; la 4,4-diméthyl-1-octyn-3-one ; la 7-chloro-1-heptyn-3-one ; la 1-hexyn-3-one ; la 1-pentyn-3-one ; la 4-méthyl-1-pentyn-3-one ; la 4,4-diméthyl-1-pentyn-3-one ; la 1-cyclohexyl-1-propyn-3-one ; le benzoacétylène et le o-chlorobenzoyl-acétylène.

Un inhibiteur **D** de type acrylonitrile peut être choisi parmi les composés de formule (D5) suivante : 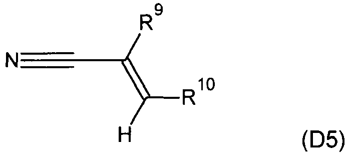 dans laquelle R⁹ et R¹⁰ représentent, indépendamment l'un de l'autre, un atome d'hydrogène, un atome de chlore, de brome ou d'iode, un groupe alkyle, un groupe cycloalkyle, un groupe (cycloalkyl)alkyle, un groupe aryle ou un groupe arylalkyle, les groupes alkyles, cycloalkyles, (cycloalkyl)alkyle, aryle ou arylalkyle pouvant éventuellement être substitué une ou plusieurs fois par un atome de chlore, de brome ou d'iode. Un inhibiteur **D** qui est un acrylonitrile utile selon l'invention peut être choisi dans le groupe constitué par les composés suivants : l'acrylonitrile ; le méthacrylonitrile ; le 2-chloroacrylonitryle ; le crotononitrile et le cinnamonitrile.

Un inhibiteur **D** de type maléate ou fumarate peut être choisi parmi les composés de formules (D6) et (D7) suivantes : 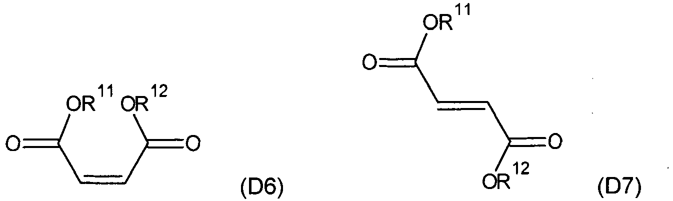 dans lesquelles R¹¹ et R¹², identiques ou différents, représentent indépendamment l'un de l'autre un groupe alkyle ou alcényle, un groupe cycloalkyle, un groupe (cycloalkyl)alkyle, un groupe aryle ou un groupe arylalkyle, lesdits groupes alkyles, alcényles, cycloalkyles, (cycloalkyl)alkyles, aryles et arylalkyles pouvant être substitués par un groupe alcoxy.
Par « alcényle », on entend selon l'invention une chaîne hydrocarbonée saturée contenant de 1 à 20 atomes de carbone, de préférence de 1 à 8 atomes de carbone comprenant au moins une double insaturation. Un groupe alcényle peut être choisi dans le groupe constitué par vinyle ou allyle.

Par « alcoxy », on entend selon les formules (D6) ou (D7), un groupe alkyle tel que défini ci-avant lié à un atome d'oxygène. Un groupe alcoxy peut être choisi dans le groupe constitué par méthoxy, éthoxy, propoxy et butoxy.

Selon un mode de réalisation particulier, R¹¹ et R¹², identiques ou différents, représentent indépendamment l'un de l'autre un groupe alkyle ou alcényle en C₁ à C₁₂, de préférence en C₁ à C₆, éventuellement substitué par un groupe alcoxy en C₁ à C₆.

Un inhibiteur **D** qui est un maléate ou un fumarate utile selon l'invention peut être choisi dans le groupe constitué par le fumarate de diéthyle, le maléate de diéthyle, le fumarate de diallyle, le maléate de diallyle et le maléate de bis-(méthoxyisopropyle).

Des inhibiteurs **D** choisis parmi les alcools α-acétyléniques, les diesters α-α'-acétyléniques, les composés conjugués ène-yne, les cétones α-acétyléniques, les acrylonitriles, les maléates, les fumarates sont disponibles dans le commerce. On peut citer notamment l'ECH (1-éthynyl-1-cyclohexanol) qui est disponible commercialement chez BASF, le maléate de diméthyle qui est disponible commercialement chez DMS et l'acétylène-dicarboxylate de diméthyle qui est disponible chez City Chemical LLC.

Ces inhibiteurs sont ajoutés en quantité en poids comprise entre 1 et 50 000 ppm par rapport au poids de la composition silicone totale, notamment entre 10 et 10 000 ppm, de préférence entre 20 et 2000 ppm et encore plus préférentiellement entre 20 ppm et 500 ppm.

Comme exemple d'additif stabilisant **K** on peut par exemple citer des dérivés silylés de l'acide phosphorique.

D'une manière préférée, l'organopolysiloxane non fonctionnalisé **I** est linéaire ou sensiblement linéaire et possède une viscosité dynamique inférieure ou égale à 50 000 mPa.s, de préférence comprise entre 20 et 40 000 mPa.s.

Selon un autre mode réalisation particulièrement avantageux, la composition silicone **X** contient au moins deux organopolysiloxanes **A** selon l'invention et caractérisée en ce que l'un au moins est une résine silicone **A1** constitué :
(i) d'au moins deux motifs siloxy de formule (A1) :

   YₐZ_{b}SiO_{(4-(a+b)/2} (A1)

   dans laquelle :
   - Y représente un groupe alcényle en C₂ à C₆, et de préférence un groupe vinyle,
   - Z représente un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle,
   - a et b représentent des nombres entiers, a valant 1, 2 ou 3, b valant 0, 1 ou 2 et (a+b) valant 1, 2 ou 3 ; et de préférence a=1 ;
(ii) et d'autres motifs siloxy de formule (A2) :

   Z_{c}SiO_{(4-C)/2} (A2)

   dans laquelle :
   - Z a la même signification que ci-dessus,
   - c représente un nombre entier valant 0, 1, 2 ou 3, et
   - avec la condition qu'au moins un motif siloxy (A2) a pour formule SiO_{4/2}.

La résine silicone **A1** est un oligomère ou polymère organopolysiloxane ramifié bien connu et disponible dans le commerce. Elle est mise en oeuvre sous forme diluée de préférence dans une huile silicone qui peut porter des fonctions vinylées. Dans ce cas le choix de l'huile silicone se fera de manière à avoir une viscosité dynamique à 25°C comprise entre 1000 mPa.s et 100000 mPa.s. Comme exemples d'oligomères ou de polymères organopolysiloxanes ramifiés on peut citer les résines « MQ », les résines « MDQ », les résines « TD » et les résines « MDT », les fonctions alcényles étant portées par les motifs siloxyles M, D et/ou T. L'homme du métier du domaine des silicones utilise couramment cette nomenclature qui représente les motifs siloxyles suivants :
- R₃SiO_{1/2} (motif M),
- RSiO_{3/2} (motif T),
- R₂SiO_{2/2} (motif D), et
- SiO_{4/2} (Motif Q).
avec R étant un groupe alcényle en C₂ à C₆ et de préférence un vinyle ou un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle.

La résine silicone **A1** particulièrement utile selon l'invention est une résine silicone à fonctions "Si-alcényle", c'est-à-dire des résines à fonctions vinyles, allyles et/ou héxényles. Avantageusement, elles comportent dans sa structure de 0,1 à 20 % en poids de groupe(s) alcényle(s). Dans cette résine, les groupements alcényles peuvent être situés sur des motifs siloxyles M, D ou T. Ces résines peuvent être préparées par exemple selon le procédé décrit dans le brevet US -A- 2 676 182. Un certain nombre de ces résines sont disponibles dans le commerce, le plus souvent à l'état de solutions, par exemple dans du xylène.

Par exemple, la résine silicone **A1** peut comprendre :
- au moins deux motifs siloxyles différents choisis parmi ceux de formules **(I)** et **(ll)** suivantes:

   WₐZ'_{b}SiO_{(4-(a+b))/2} **(I)**

   dans laquelle :
   - les symboles W, identiques ou différents, représentent chacun un groupe alcényle en C₂-C₆; de préférence un groupe vinyle, allyle et/ou héxényle et encore plus préférentiellement un groupe vinyle,
   - les symboles Z', identiques ou différents, représentent chacun un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle, et
   - le symbole a est égal à 1 ou 2, de préférence 1, le symbole b est égal à 0, 1 ou 2 et la somme a + b est égale à 1, 2 ou 3,
- et éventuellement des motifs de formule suivante:

   Z'_{c}SiO_{(4-c)/2} **(II)**

   dans laquelle Z' a la même signification que ci-dessus et le symbole c est égal à 0, 1, 2 ou 3, et
avec le condition qu'au moins un des motifs **(I)** ou **(II)** est un motif T ou Q.

Par motif « T » on entend un motif siloxyle de formule RSiO_{3/2} et par motif « Q » on entend un motif siloxyle de formule SiO_{4/2} et les groupements R sont tels que décrits ci-dessous.

Dans un mode préféré de réalisation de l'invention, la résine silicone **A1** est choisie parmi le groupe constitué par les résines silicones suivantes :
- MD^{Vi}Q où les groupes vinyles sont inclus dans les motifs D,
- MD^{Vi}TQ où les groupes vinyles sont inclus dans les motifs D,
- MM^{Vi}Q où les groupes vinyles sont inclus dans une partie des motifs M,
- MM^{Vi}TQ où les groupes vinyles sont inclus dans une partie des motifs M,
- MM^{Vi}DD^{Vi}Q où les groupes vinyles sont inclus dans les motifs M et D,
- et leurs mélanges,
avec :
- M = motif siloxyle de formule R₃SiO_{1/2}
- M^{Vi} = motif siloxyle de formule (R₂)(vinyle)SiO_{1/2}
- D = motif siloxyle de formule R₂SiO_{2/2}
- D^{Vi} = motif siloxyle de formule (R)(vinyle)SiO_{2/2}
- Q = motif siloxyle de formule SiO_{4/2} ;
- T = motif siloxyle de formule RSiO_{3/2}, et
- les groupements R, identiques ou différents, sont des groupements hydrocarbonés monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle.

Selon un autre mode particulier de l'invention, la résine silicone **A1** est additionnée à la composition selon l'invention sous forme d'un mélange dans au moins une huile organopolysiloxane, par exemple correspondant à la définition de l'organopolysiloxane **A** décrit ci-dessus, ou dans au moins un solvant hydrocarboné tels que le toluène, le xylène ou des dérivés connus sous l'appellation Exxsol® et vendus par la société Exxon Mobil.

De préférence la résine silicone **A1** comportant au moins deux radicaux alcényles en C₂ à C₆ liés chacun à un atome de silicium a pour formule MD^{Vi}Q dans laquelle:
- M est un motif siloxyle monovalent de formule R₃SiO_{1/2}
- D^{Vi} est un motif siloxyle divalent de formule RXSiO_{2/2}, et
- Q est un motif siloxyle tétravalent de formule SiO_{4/2},
- avec le radical X correspondant à un groupement alcényle ayant de 2 à 6 atomes de carbone, de préférence un groupement vinyle, et
- le radical R correspondant à un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle.

Selon un autre mode de réalisation de l'invention, la résine silicone **A1** est présente dans la composition **X** jusqu'à 90 % en poids par rapport au poids total de la composition **X,** de préférence entre 10 et 90% en poids par rapport au poids total de la composition **X** et de manière encore plus préférée entre 30 et 70% en poids par rapport au poids total de la composition **X .** Pour un usage optimum, la résine silicone **A1** est présente dans la composition **X** entre 40 et 60% en poids par rapport au poids total de la composition **X.**

On peut citer comme exemple de résine silicone **M** celles qui ont :
- au moins deux motifs siloxyles différents choisis parmi ceux de formules **(III)** et **(IV)** suivantes:

   W'ₐZ'_{b}SiO_{(4-(a+b))/2} **(III)**

   dans laquelle :
   - les symboles W' représentent un groupe hydroxyle (-OH),
   - les symboles Z', identiques ou différents, représentent chacun un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle, et
   - le symbole a est égal à 1 ou 2, de préférence égal à 1, le symbole b est égal à 0, 1 ou 2 et la somme a + b est égale à 1, 2 ou 3,
- et éventuellement des motifs de formule suivante:

   Z'_{c}SiO_{(4-c)/2} **(IV)**

   dans laquelle Z' a la même signification que ci-dessus et le symbole c est égal à 0, 1, 2 ou 3, et
avec le condition qu'au moins un des motifs **(III)** ou **(IV)** est un motif T ou Q.

La résine silicone **M** ayant des groupements hydroxyles et exempt de groupement alcényles ou hydrogénosilyles SiH est une résine silicone à fonctions hydroxyle (-OH) avec Z' étant un groupe alkyle en C₁ à C₈. Des exemples de groupes R' sont les groupes : méthyle, éthyle, propyle, butyle,.... Ces résines organosiliciques sont préparées par cohydrolyse et cocondensation de chlorosilanes. Ces résines sont des oligomères ou polymères organopolysiloxanes ramifiés bien connus et disponibles dans le commerce. Elles présentent, dans leur structure, au moins deux motifs siloxyles différents choisis parmi ceux de formule (Z')₃SiO_{1/2} (motif M), (Z¹)₂SiO_{2/2} (motif D), Z'SiO_{3/2} (motif T) et SiO_{4/2} (motif Q), l'un au moins de ces motifs étant un motif T ou Q. Les symboles Z' ont la même définition que ci-dessus. Ces résines ne sont pas complètement condensées et elles possèdent une teneur pondérale en groupes hydroxyles comprise entre 0,2 et 10 % en poids ou une teneur pondérale en groupe hydroxyle comprise entre 5 et 500 meq/100 g de résine.

Selon un autre mode de réalisation particulier de l'invention, la résine silicone **M** est présente dans la composition **X** à une teneur comprise entre 1 et 45 % en poids par rapport au poids total de la composition **X,** de préférence entre 2 et 45% en poids par rapport au poids total de la composition **X** et de manière encore plus préférée entre 2 et 35 % en poids par rapport au poids total de la composition **X** .

Le film souple en polyuréthane peut-être fabriqué à partir de polyuréthane fondu soufflé.

Préférentiellement, on utilise un film souple en polyuréthane transparent ou translucide. Lorsque l'article adhésif a un usage de pansement, l'utilisation d'un film transparent ou translucide présente l'avantage de permettre l'observation de la plaie, de la blessure ou du site d'entrée d'un cathéter sur lequel le pansement doit être centré.

De préférence, le dit support **S** est un film souple en polyuréthane ayant une épaisseur de 5 à 600 µm, de préférence de 5 à 250 µm et encore plus préférentiellement de 10 à 100 µm.

Comme exemple de film souple en polyuréthane on peut citer ceux qui sont utilisés dans les pansements commercialisés par la société Smith&Nephew sous la marque Opsite®, ou par la société 3M sous la marque Tegaderm® ou encore par les Laboratoires URGO sous la marque Optiskin®. Ces pansements sont constitués d'un film mince (de l'ordre de 20 à 50 µm) transparent de polyuréthane adhésivé. Leur transparence permet un contrôle visuel de la zone à traiter.

Comme autre exemple de film souple en polyuréthane on peut aussi citer ceux commercialisés sous les marques Platilon® vendus par la société Bayer MaterialScience et Inspire® vendus par la société Coveris Advanced Coatings.

Selon un mode de réalisation préféré, le support **S** est un film souple continu qui est perméable à l'air et imperméable aux fluides.

Le film peut présenter un taux de transmission de vapeur d'eau (MVTR : Moisture Vapor Transmission Rate) variable suivant l'application visée. Une technique de mesure du taux de transmission de vapeur d'eau en contact liquide est décrite dans la norme NF-EN 13726-2. De préférence, le film souple en polyuréthane sera choisi de manière à obtenir un pansement ayant un taux de transmission de vapeur d'eau (MVTR : Moisture Vapor Transmission Rate) supérieur à 300 g/m²/24 heures, de préférence supérieur ou égal à 600 g/m²/24 heures, de préférence encore supérieur ou égal à 1000 g/m²/24 heures.

Selon un autre mode de réalisation particulier, l'invention concerne un article adhésif sur la peau caractérisé en ce que le support **S** qui est un film souple continu en polyuréthane comporte sur au moins une partie de la face de dessous **S2** un adhésif sensible à la pression. Selon une variante avantageuse de l'invention le film souple continu en polyuréthane est perforé de manière à pouvoir favoriser la circulation des exsudats.

Ainsi l'article adhésif selon l'invention est selon un mode de réalisation particulier un stratifié adhésif amovible et présente l'avantage de pouvoir être utilisé en tant que couche de contact avec la peau dans différents types de pansements.

L'adhésif sensible à la pression peut être n'importe lequel des nombreux adhésifs sensibles à la pression connus de l'art. Ces adhésifs, généralement sous une forme anhydre et sans solvant, sont collants de façon permanente à température ambiante et adhèrent fermement à une variété de surfaces dissemblables lors d'un contact simple, sans qu'il y ait besoin d'utiliser plus que la pression d'un doigt ou de la main. Ils ne nécessitent pas d'activation par de l'eau, un solvant ou de la chaleur afin d'avoir une forte force adhésive de maintien. Des exemples d'adhésifs sensibles à la pression comprennent les adhésifs de caoutchouc/résine, qui sont des mélanges de matière caoutchouteuse et de résine dure, et les adhésifs acryliques (ou d'acrylate). La classe actuellement préférée d'adhésifs sensibles à la pression pour une utilisation dans la présente invention est celle des adhésifs acryliques.

Selon un mode de réalisation particulier, le support **S** est un film souple perforé en polyuréthane ou un film souple continu en polyuréthane ayant une face de dessus **S1** et une face de dessous **S2** et qui est imperméable à l'air et aux fluides dans ses parties comprises entre les perforations.

Dans ce mode de réalisation particulier, il est avantageux que les perforations du support S soient circulaires et aient un diamètre de 50 µm à 10 mm.

Selon un autre mode de réalisation particulier, l'article adhésif sur la peau selon l'invention est caractérisé en ce qu'il comprend une ou plusieurs couches **N** comprenant un corps absorbant **O,** éventuellement séparées par une ou plusieurs couches intermédiaires **P,** placée(s) sur le support **S** du côté de la face de dessous **S2** du support **S.** De préférence, le corps absorbant **O** est choisi parmi le groupe constitué par: une mousse hydrophile, un tampon en tissu, un hydrogel, un hydrocolloïde et un alginate. De préférence, le corps absorbant **O** est une mousse en polyuréthane.

La couche **D1** est constituée par un gel silicone **E** obtenu par réticulation d'une composition silicone **Y** réticulable par une réaction d'hydrosilylation. Les gels de silicone sont couramment employés dans le domaine médical que ce soit à usage externe (prothèses mammaires, coussins ou matelas médicaux) ou à usage interne (prothèses mammaires placées in situ, pansements). Ils sont facilement manipulables et ont de bonnes propriétés mécaniques, leur densité étant proche de celle des tissus humains. Au sens de la présente invention, l'expression « gel silicone » désigne un produit silicone réticulé caractérisé notamment par un taux de pénétration (ou « pénétrabilité ») compris entre 50 et 500 dixièmes de mm et de préférence entre 80 et 300 dixième de mm (mesure par pénétrométrie NF ISO 2137, pénétromètre modèle PNR 12 Petrotest, poids de la tige et du cône: 62,5 g). La pénétrabilité au cône d'un gel silicone est déterminée à 25 °C en mesurant la profondeur de pénétration du cône dans l'échantillon, celle-ci étant obtenue en libérant l'ensemble cône du pénétromètre et en laissant le cône agir pendant 5 s. Le gel silicone présente une cohésion telle qu'il ne laisse pas de résidus sur la peau. Il peut être préparé à partir de précurseurs de silicones qui réticulent après leur mise en contact suivant une réaction d'hydrosilylation. De tels systèmes sont connus de l'art antérieur, par exemple dans les documents EP-A-0 251 810, EP-A-0 300 620 ou US-A-4 921 704. Les compositions décrites se présentent sous forme bicomposante et la réticulation s'effectue après mélange de deux parties nommées A et B. Les mélanges de précurseurs décrits dans ces documents comprennent pour l'essentiel :
- une partie A qui comprend un polydiméthylsiloxane substituée par au moins deux groupes vinyle, le plus souvent situés à chacune de ses extrémités et un catalyseur de platine, et
- une partie B qui comprend un polydiméthylsiloxane ayant au moins deux groupes hydrogénosilanes et le plus souvent au moins trois groupes hydrogénosilanes.

La mise en présence des deux parties provoque, en présence du catalyseur au platine, une réaction de réticulation (par une réaction de polyaddition et en particulier une réaction d'hydrosilylation) des deux polydiméthylsiloxanes fonctionnalisées qui se produit avantageusement à température ambiante et peut être accélérée par la chaleur. Des additifs comme des pigments, des inhibiteurs ou des charges de remplissage peuvent être incorporés à l'une au moins des deux parties. Des exemples de compositions précurseurs du gel de silicone adhésif peuvent être choisis parmi les produits suivants : Silbione® HC2 2011 A&B, Silbione® HC2 2031 A&B, Silbione® HC2 2022 A&B, Silbione® RT Gel 4642 A&B, Silbione® RT Gel 4712 A&B, Silbione® RT Gel 4317 A&B et Silbione® RT Gel4717 A&B de la société Bluestar Silicones, ceux de la gamme Silpuran® de Wacker-Chemie GmbH, Nusil® MED- 6340, Nusil® MED-6345 de Nusil Technology, et Dow Corning® MG 7-9800®, Dow Corning® MG 7-9850, Dow Corning® MG 7-9900® Soft Skin Adhesives Parts A&B de Dow Corning Corp.

De préférence, les quantités de gel silicone seront déterminées de manière à obtenir des enductions ayant une teneur en gel silicone comprise entre 20 et 500 g/m² de support, de préférence entre 40 et 250 g/m² et encore plus préférentiellement entre 80 et 350 g/m².

De préférence, le gel silicone **E** est préparé par réticulation d'une composition silicone **K** comprenant :
- au moins un organopolysiloxane **G** ayant en moyenne par molécule deux groupes alcényles liés chacun à atome de silicium, lesdits groupes alcényle comptant chacun 2 à 6 atomes de carbone et aucun atome de silicium n'étant lié à plus d'un seul groupe alcényle,
- au moins un composé hydrogéné du silicium **H** ayant par molécule au moins deux et de préférence au moins trois atomes d'hydrogène liés chacun à un atome de silicium,
- éventuellement au moins un organopolysiloxane non fonctionnalisé **I** et
- un catalyseur d'hydrosilylation **J** à base de platine.

En général, l'organopolysiloxane **G** contient en moyenne deux groupes alcényle liés à du silicium par molécule, chaque groupe alcényle étant lié à un atome de silicium différent. L'organopolysiloxane **G** est un polymère sensiblement linéaire, bien qu'un faible degré de ramification puisse exister. De préférence, les groupes alcényle sont fixés à des atomes de silicium, et de préférence ils sont fixés aux atomes de silicium terminaux de la chaîne siloxane. Les groupes alcényle comptent au maximum 6 atomes de carbone et il peut s'agir par exemple de groupes vinyle, allyle ou hexényle, bien que ce soient de préférence des groupes vinyle. Les substituants organiques restants de l'organopolysiloxane **G** sont choisis parmi les groupes alkyle et aryle, s'agissant de préférence de groupes alkyle n'ayant pas plus de 8 atomes de carbone et de groupes phényle. Des exemples de ces substituants restants sont les groupes méthyle, éthyle, propyle, isobutyle et phényle. Les plus volontiers mis en oeuvre sont des polydiméthylsiloxanes α,ω(diméthyvinylsiloxy) ou des polyorganosiloxanes de type poly(diméthylsiloxy)(méthylvinylsiloxy)α,ω(diméthylvinylsiloxy).

L'organopolysiloxane **G** est un produit commercial comme par exemple les produits de la gamme BLUESIL® 621V de la société Bluestar Silicones, et sont largement divulgués tant en ce qui concerne leurs structures que leurs synthèses dans la littérature technique.

D'une manière préférée, le polyorganosiloxane **G** est linéaire ou sensiblement linéaire et possède une viscosité dynamique inférieure ou égale à 200 000 m.Pa.s, de préférence inférieure ou égale à 170000 mPa.s et encore plus préférentiellement comprise entre 20 et 165000 mPa.s.

Selon une autre variante, le % en poids de groupes réactifs alcényles liés directement à un atome de silicium, est compris entre 0,025 % et 3%.

Le composé hydrogéné du silicium **H** est en général un polyorganosiloxane, ou un silane, comportant par molécule au moins deux, de préférence trois, atomes d'hydrogène liés à du silicium. Ces atomes d'hydrogène peuvent être situés sur des motifs siloxanes terminaux et également sur des motifs siloxanes se trouvant dans la chaîne polymère, ou bien ils peuvent être situés uniquement dans la chaîne siloxane.

En pratique, les polyorganohydrogénosiloxanes **H** mis en oeuvre sont par exemple les polyorganosiloxanes de type poly(diméthylsiloxy)-(siloxyméthylhydrogéno)-α,ω-(diméthylhydrogénosiloxy) et les polydiméthylsiloxanes-α,ω-(diméthylhydrogénosiloxy). Ce sont des produits commerciaux et sont largement divulgués tant en ce qui concerne leurs structures que leurs synthèses dans la littérature technique.

Le catalyseur **J** a la même définition que le catalyseur **C** exposé ci-dessus. Pour le catalyseur **J,** on entend par quantité efficace d'au moins un catalyseur de réaction d'hydrosilylation, la quantité suffisante pour amorcer la réaction d'hydrosilylation. Concernant la quantité catalytiquement efficace à mettre en oeuvre, il va de soi que l'homme du métier du domaine considéré est parfaitement à même de déterminer la quantité optimale de catalyseur pour promouvoir la réaction d'hydrosilylation . Cette quantité dépend notamment de la nature du catalyseur et des organopolysiloxanes en cause. Pour fixer les idées on peut indiquer qu'elle sera comprise entre 0,001 et 0,5 % en poids par rapport au poids total de la composition.

De préférence, les quantités des constituants **G et H** sont choisies de manière à ce que le rapport molaire r des atomes d'hydrogène liés au silicium aux radicaux alcényles (X) liés au silicium soit compris entre 0,5 :1 et 2 :1.

S'agissant de la préparation du gel, on peut préciser que la réticulation de la composition en gel intervient à la température ambiante ou après chauffage à des températures comprises entre 50 et 200° C par exemple. Dans ce contexte, les durées de réticulation nécessaires sont, par exemple, de quelques minutes. Ces produits sont largement diffusés et décrits dans le commerce et sont bien connus de l'homme du métier.

La couche de protection **F** constituée d'un matériau de protection pelable peut être constitué d'une ou plusieurs parties pelables avant usage. Cette couche de protection recouvre de préférence toute la surface du pansement et pourra être de tout matériau couramment utilisé comme protection par l'homme du métier dans le domaine des pansements. Elle pourra se présenter par exemple sous forme de film par exemple un film de polyoléfine tels que le polyéthylène ou le polypropylène ou un film de polyester. Ce film peut-être avantageusement traité sur au moins l'une de ses faces par un composé siliconé comme un silane, un composé fluoré, ou un composé siliconé et fluoré. Le choix du matériau est en général adapté à la nature du gel de silicone. La couche de protection **F** constituée d'un matériau de protection pelable est de préférence doté d'une épaisseur comprise entre 10 et 100 µm, par exemple de l'ordre de 50 µm.

Selon un autre mode de réalisation de l'invention, l'article adhésif sur la peau selon l'invention est caractérisé en ce que :
- le support **S** est un film souple perforé en polyuréthane, ayant une face de dessus **S1** et une face de dessous **S2**,
- le primaire d'accrochage **C1** est sur la face de dessus **S1** dudit support **S** et a été appliqué de façon discontinue de manière à empêcher le bouchage des perforations du support **S**, et
- la couche **D1** constituée par un gel silicone **E** a été appliquée de façon discontinue de manière à empêcher le bouchage des perforations du support **S.**

Une technique pour empêcher le bouchage des perforations est connue par l'homme du métier qui pourra se référer aux brevets EP-633758 ou EP633757 qui préconisent l'utilisation d'air (froid ou à une température inférieure ou égale à 20°C) qui est soufflé sur la face de dessous du support perforé de manière à s'écouler dans les perforations du support de manière à chasser les compositions silicones (avant leur stade de réticulation) qui obstruent les perforations. Ensuite la température de l'air soufflée est augmentée de manière à démarrer le durcissement des compositions silicones situés juste autour des perforations. Le matériau de support est imperméable à l'air ou suffisamment imperméable pour que sensiblement tout l'air soufflé s'écoule par les perforations. Il est aussi possible d'utiliser un matériau support permettant à l'air de diffuser à travers celui-ci.

Selon un mode de réalisation particulier l'article adhésif sur la peau selon l'invention est caractérisé en ce que :
- le primaire d'accrochage **C1** est appliqué sur au moins une partie ou sur la totalité de la face de dessus **S1** et sur une partie ou sur la totalité de la face de dessous **S2** dudit support **S**, et
- au moins une couche **D1** constituée par un gel silicone **E** obtenu par réticulation d'une composition silicone **Y** réticulable par une réaction d'hydrosilylation a été appliquée sur ledit primaire d'accrochage **C1** présent sur la face de dessus **S1** et sur la face de dessous **S2** dudit support **S.**

De préférence, l'épaisseur du support **S** est comprise entre 5 et 200 µm, de préférence entre 10 et 75 µm.

Selon un mode de réalisation particulier, l'article adhésif sur la peau selon l'invention est caractérisé en ce qu'il s'agit d'un pansement à usage médical ou paramédical.

Un autre objet de l'invention concerne un procédé de préparation d'un article adhésif sur la peau selon l'invention et tel que décrit ci-dessus caractérisé en ce qu'il comprend :
- une première étape consistant à déposer sur ledit support **S** des quantités suffisantes de la composition silicone **X** selon l'invention et telle que décrite ci-dessus et à faire réticuler la composition silicone **X,** de préférence à une température comprise entre 60 et 200°C, pendant un temps suffisant de manière à former ledit primaire d'accrochage **C1**,
- une deuxième étape consistant à déposer sur ledit primaire d'accrochage **C1** des quantités suffisantes de la composition silicone **Y** selon l'invention et telle que décrite ci-dessus et à faire réticuler la composition silicone **Y,** de préférence à une température comprise entre 60 et 200°C, pendant un temps suffisant de manière à former la dite couche **D1.**

Selon une variante avantageuse, l'invention concerne aussi un procédé de préparation d'un article adhésif sur la peau selon l'invention et tel que décrit ci-dessus caractérisé en ce qu'il comprend :
- une étape consistant à déposer de façon successive sur ledit support S des quantités suffisantes:
   - de la composition silicone **X** selon l'invention et telle que décrite ci-dessus, et
   - puis ensuite de la composition silicone **Y** selon l'invention et telle que décrite ci-dessus sur la composition silicone **X,**
puis de faire réticuler lesdites compositions silicones **X** et **Y,** de préférence à une température comprise entre 60 et 200°C, pendant un temps suffisant de manière à former ledit primaire d'accrochage **C1** et la dite couche **D1.**

De préférence, les quantités de la composition silicone **X** appliquées sur le support **S** seront déterminées de manière à obtenir des enduction ayant une teneur en composition silicone **X** avant réticulation comprise entre 1 et 100 g/m² de support, de préférence entre 1 et 50 g/m² et encore plus préférentiellement entre 2 et 30 g/m².

De préférence, les quantités de la composition **Y** seront déterminées de manière à obtenir des enductions ayant une teneur en gel silicone **E** comprise entre 20 et 500 g/m² de support, de préférence entre 40 et 250 g/m² et encore plus préférentiellement entre 80 et 350 g/m².

Comme technique pour déposer les compositions **X** et **Y,** on peut citer par exemple les techniques d'enduction réalisées par racle, en particulier par racle sur cylindre, racle en l'air et racle sur tapis, ou par foulardage, c'est-à-dire par exprimage entre deux rouleaux, ou encore par rouleau lécheur, cadre rotatif, rouleau inverse "reverse roll", transfert, pulvérisation.

Comme autre technique d'enduction on peut citer la technique de couchage au rideau. Le couchage au rideau est un procédé d'application d'un liquide de couchage sur un article ou un support. Le couchage au rideau est caractérisé par la formation d'un rideau tombant librement d'un liquide de couchage qui tombe de la lèvre du Hopper et, sous l'effet de la gravité, vient rencontrer l'article se déplaçant à travers le rideau pour former un couchage (ou une enduction). Cette technique a été très utilisée dans le domaine de la préparation de supports argentiques photosensibles multicouches (voir par exemple les brevets US-3508947, US3508947 ou EP537086).

Il est connu que la qualité du couchage dépend de la qualité du rideau tombant librement. Il est préférable que le rideau présente un écoulement laminaire stable depuis l'endroit où il se forme jusqu'à la ligne rencontre avec le support en mouvement. A défaut, la tension superficielle amènera le rideau à se contracter vers l'intérieur et à interrompre l'écoulement laminaire. Afin d'éviter ce problème, il est connu d'employer des guides de bord pour saisir le rideau tombant librement au niveau de ses bords et empêcher sa contraction vers l'intérieur en raison de la tension superficielle. Des exemples de tels systèmes sont décrits dans les brevets US4933215, US4479987, US4974533, US3632374, US4479987, EP537086 et US4830887.

L'avantage du couchage au rideau est qu'il peut permettre l'enduction en simultanée des compositions silicones **X** et **Y** selon l'invention.

Selon un mode de réalisation avantageux, le procédé selon l'invention est caractérisé en ce que pour ladite composition silicone **X** les quantités en poids des organopolysiloxanes **A** et **B** sont déterminées de manière à ce que le rapport RHalk =1 avec RHalk = nH/tAlk et :
- nH = nombre de mole d'atome d'hydrogène directement lié à un atome de silicium de l'organopolysiloxane **B,** et
- tAlk = nombre de mole d'alcényle directement lié à un atome de silicium de l'organopolysiloxane **A.**

Ce mode de réalisation particulier est particulièrement avantageux lorsque l'on dépose de façon successive sur ledit support **S** des quantités suffisantes:
- de la composition silicone **X** selon l'invention et telle que décrite ci-dessus, et
- puis ensuite de la composition silicone **Y** selon l'invention et telle que décrite ci-dessus sur la composition silicone **X, et**
   alors on effectue l'étape de réticulation.

Cette variante permet de maintenir un tack suffisamment important pour le gel de silicone **E** résultant de l'enduction et de la réticulation de la composition silicone **Y** précurseur du gel silicone.

Les exemples non limitatifs qui suivent, montrent diverses possibilités de formulation des compositions selon l'invention ainsi que les caractéristiques et les propriétés des gels silicones obtenus par réticulation desdites compositions.

Un autre objet de l'invention concerne un pansement ou patch à usage médical ou paramédical comprenant l'article adhésif sur la peau selon l'invention et tel que décrit ci-dessus.

### EXEMPLES

Les compositions décrites se présentent sous la forme bicomposante. La réticulation du primaire s'effectue après mélange à 25°C, de deux parties nommées Partie A et Partie B dans un rapport 50 / 50. La réticulation du gel Silbione® HC2 2022 dans les exemples ci-dessous s'effectue après mélange, à 25°C, de deux parties dans un rapport 50 / 50.

### 1) Matières premières utilisées dans la composition du primaire selon l'invention

POS(A1) = résine poly(diméthylsiloxy) (méthylvinylsiloxy) et contenant des motifs siloxyles tétravalents (motifs Q).
POS(A2) = huile polydiméthylsiloxane α,ω(dimethylvinylsiloxy) de viscosité 600mPa.s.
POS(B) = huile poly(diméthylsiloxy)(méthylhydrogénosiloxy)α,ω diméthylhydrogénosiloxy de viscosité 25mPa.s et contenant 0.7% en poids de groupement H.
(C) = complexe organométallique du platine utilisé comme catalyseur de la réaction
(D) = inhibiteur de la réaction d'hydrosilylation de type alcool α-acétylénique
(K) = stabilisant du mélange

### 2) Le Tableau 1 décrit les concentrations de chacun de ces constituants dans les mélange Partie A + Partie B testés.

**TABLEAU 1**

| Constituants | Mélange A1+B1 (comparatif) | Mélange A2+B2 (invention) | Mélange A3+B3 (invention) | Mélange A4+B4 (comparatif) |
|---|---|---|---|---|
| POS(A1) | 52,32 | 52,13 | 50,21 | 43,23 |
| POS(A2) | 45,12 | 45,12 | 45,12 | 45,12 |
| POS(B) | 2,50 | 2,69 | 4,60 | 11,59 |
| (C) | 0,020 | 0,020 | 0,020 | 0,020 |
| (D) | 0,020 | 0,020 | 0,020 | 0,020 |
| (K) | 0,020 | 0,020 | 0,020 | 0,020 |
| | | | | |
| RHalk | 0,96 | 1 | 1,8 | 5 |

### 2) Méthodologies d'obtention des enductions testées

Deux méthodologies différentes ont été suivies pour tester les compositions ci-dessus comme primaire permettant l'amélioration de l'adhésion du gel Silbione® HC2 2022 sur un support qui est un film souple en polyuréthane.

### Méthodologie d'enduction du primaire puis du gel, sans réticulation préalable du primaire

- Application du primaire à un poids compris entre 5 et 15g/m² sur un support qui est un film souple en polyuréthane. Le primaire est appliqué sur le film à l'aide d'une racle d'enduction, puis
- application du gel Silbione® HC2 2022 à un poids de 200g/m² à l'aide d'une racle d'enduction, et
- réticulation du composite pendant 30 min à 120°C en étuve ventilée.

### Méthodologie d'enduction du primaire puis du gel, avec réticulation préalable du primaire

- Application du primaire à un poids compris entre 5 et 15g/m² sur un support qui est un film souple en polyuréthane. Le primaire est appliqué sur le film à l'aide d'une racle d'enduction, puis
- réticulation du primaire pendant 5 min à 150°C en étuve ventilé, puis.
- application du gel Silbione® HC2 2022 à un poids de 200g/m² à l'aide d'une racle d'enduction, et
- réticulation du composite pendant 30 min à 120°C en étuve ventilée

### 3)Tests réalisés sur les enductions

Les deux propriétés clés testées pour évaluer l'efficacité du primaire sont l'adhésion du primaire ou du gel au support souple en polyuréthane et le pouvoir adhésif du gel sur la peau (tack). En effet, lorsque l'adhésion d'un gel silicone sur un film souple en polyuréthane est améliorée par l'utilisation d'un primaire, il est important de s'assurer que le pouvoir adhésif du gel sur la peau (tack) est conservé et que l'adhésion d'un dispositif médical à la peau ne serait pas altérée par l'utilisation d'un primaire.

### Evaluation du pouvoir adhésif du gel silicone sur la peau

Le PROBE TACK Device (PT-1000) est l'appareil utilisé pour évaluer le pouvoir adhésif du gel enduit. Le test est réalisé selon la norme ASTM D2979 avec les paramètres de test suivants :
- surface de contact avec l'adhésif : 0.2cm²,
- temps de contact avec l'adhésif : 1s,
- pression de contact : 20 gf,
- vitesse de séparation du poinçon de l'adhésif : 10mm/s.

L'énergie de décollement du substrat est exprimée en mJ/cm².

### Evaluation de l'adhésion du gel silicone au support qui est un film souple en polyuréthane

L'adhésion du gel silicone au support qui est un film souple en polyuréthane consiste en une évaluation qualitative de la résistance du gel lors de gommages au doigt. Le gel sera considéré comme plus adhérent au film polyuréthane si le nombre de gommage (aussi nommé Rub off) est plus important avant d'observer la délamination du gel de son substrat.

### 4) Les Tableaux 2 et 3 présentent les propriétés d'application obtenues en fonction de la méthodologie d'enduction employée

### a) Selon la méthodologie d'enduction du primaire puis du gel, sans réticulation préalable du primaire :

**TABLEAU 2**

| | Sans primaire | Avec primaire | | | |
|---|---|---|---|---|---|
| | | Mélange A1+B1 (comparatif) | Mélange A2+B2 (invention) | Mélange A3+B3 (invention) | Mélange A4+B4 (comparatif) |
| Probe Tack (mJ/cm²) | 20 | 20 | 21 | 14 | 11 |
| Adhésion du gel sur film polyuréthane (nombre de gommage au doigt) | 5 | 5 | >30 | >30 | >30 |

Le gel enduit sur primaire selon ce premier procédé présente :
a) une bonne adhésion sur un support qui est un film souple en polyuréthane, et
b) une adhésion à la peau satisfaisante,
dans la mesure où le ratio RHalk du primaire est supérieur ou égal à 1 et inférieur ou égal à 4,5.

La composition du primaire dont le ratio RHalk est égal à 1 (mélange A2 + B2) apporte un très bon compromis de propriétés (amélioration de l'adhésion du gel silicone au support souple en polyuréthane, conservation du pouvoir adhésif et le tack du gel sur la peau).

### b) Selon la méthodologie d'enduction du primaire puis du gel, avec réticulation préalable du primaire

**TABLEAU 3**

| | Sans primaire | Avec primaire | | | |
|---|---|---|---|---|---|
| | | Mélange A1+B1 (comparatif) | Mélange A2+B2 (invention) | Mélange A3+B3 (invention) | Mélange A4+B4 (comparatif) |
| Probe Tack (mJ/cm²) | 20 | 19 | 19 | 21 | 11 |
| Adhésion du gel sur film polyuréthane (nombre de gommage au doigt) | 5 | 5 | 20 | >30 | >30 |

Le gel enduit sur primaire selon ce deuxième procédé présente :
a) une bonne adhésion sur un support qui est un film souple en polyuréthane, et
b) une adhésion à la peau satisfaisante,
dans la mesure où le ratio RHalk du primaire est supérieur ou égal à 1 et inférieur ou égal à 4,5.

La composition du primaire dont le ratio RHalk est égal à 1,8 (mélange A3 + B3) apporte un très bon compromis de propriétés (amélioration de l'adhésion du gel silicone au support souple en polyuréthane, conservation du pouvoir adhésif, le tack et du gel sur la peau).

## Revendications

1. Article adhésif sur la peau comprenant :
• un support **S** qui est un film souple en polyuréthane ayant une face de dessus **S1** et une face de dessous **S2,**
• au moins un primaire d'accrochage **C1** appliqué sur au moins une partie ou sur la totalité de la face de dessus **S1** dudit support **S**,
• au moins une couche **D1** constituée par un gel silicone **E** obtenu par réticulation d'une composition silicone **Y** réticulable par une réaction d'hydrosilylation qui a été appliquée sur ledit primaire d'accrochage **C1** à l'opposé dudit support **S**, et
• éventuellement au moins une couche de protection **F** constituée d'un matériau de protection pelable et appliquée sur ladite couche **D1,**
• ledit primaire d'accrochage **C1** étant **caractérisé en ce qu'**il est constitué d'un élastomère silicone qui n'est pas un gel silicone et qui est obtenu par réticulation d'une composition silicone **X** constituée de:
1) au moins un organopolysiloxane **A** comportant, par molécule, au moins deux radicaux vinyles liés chacun à un atome de silicium et constitué :
(i) d'au moins deux motifs siloxy de formule (A1) :
YₐZ_{b}SiO_{(4-(a+b)/2} (A1)
dans laquelle :
- Y représente un groupe alcényle en C₂ à C₆, et de préférence un groupe vinyle,
- Z représente un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle,
- a et b représentent des nombres entiers, a valant 1, 2 ou 3, b valant 0, 1 ou 2 et (a+b) valant 1, 2 ou 3 ;
(ii) et comportant éventuellement d'autres motifs siloxy de formule (A2) :
Z_{c}SiO_{(4-c)/2} (A2)
dans laquelle :
- Z a la même signification que ci-dessus, et
- c représente un nombre entier valant 0, 1, 2 ou 3.
2) au moins un organopolysiloxane **B** comportant, par molécule, au moins trois atomes d'hydrogène liés chacun à un atome de silicium et constitué :
(i) d'au moins trois motifs siloxy de formule (B1) :
HZ_{b}SiO_{(3-b)/2} (B1)
dans laquelle :
- H est un atome d'hydrogène,
- Z représente un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle,
- b valant 0, 1 ou 2;
(ii) et comportant éventuellement d'autres motifs siloxy de formule (B2) :
Z_{c}SiO_{(4-c)/2} (B2)
dans laquelle :
- Z a la même signification que ci-dessus, et
c représente un nombre entier valant 0, 1, 2 ou 3.
3) au moins un catalyseur **C** d'hydrosilylation,
4) au moins un inhibiteur **D** de la réaction d'hydrosilylation,
5) éventuellement au moins un additif stabilisant **K,** et
7) éventuellement au moins une résine silicone **M** ayant des groupements hydroxyles et exempt de groupement alcényles ou hydrogénosilyles SiH, et
- avec la condition que les quantités en poids des organopolysiloxanes **A** et **B** sont déterminées de manière à ce que le rapport RHalk = nH/tAlk et 1,00 ≤ RHalk ≤ 4,5 avec :
- nH = nombre de mole d'atome d'hydrogène directement lié à un atome de silicium de l'organopolysiloxane **B,** et
- tAlk = nombre de mole d'alcényle directement lié à un atome de silicium de l'organopolysiloxane **A.**

2. Article adhésif sur la peau selon la revendication 1 **caractérisé en ce que** les quantités en poids des organopolysiloxanes **A** et **B** sont déterminées de manière à ce que 1,50 ≤ RHalk ≤ 4,0.

3. Article adhésif sur la peau selon la revendication 1 dans lequel la composition silicone X contient au moins deux organopolysiloxanes A selon là revendication 1 et **caractérisé en ce que** l'un au moins est une résine silicone **A1** constitué :
(i) d'au moins deux motifs siloxy de formule (A1) :
YₐZ_{b}SiO_{(4-(a+b)/2} (A1)
dans laquelle :
- Y représente un groupe alcényle en C₂ à C₆, et de préférence un groupe vinyle,
- Z représente un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle,
- a et b représentent des nombres entiers, a valant 1, 2 ou 3, b valant 0, 1 ou 2 et (a+b) valant 1, 2 ou 3 ; et de préférence a=1 ;
(ii) et d'autres motifs siloxy de formule (A2) :
Z_{c}SiO_{(4-c)/2} (A2)
dans laquelle :
- Z a la même signification que ci-dessus,
- c représente un nombre entier valant 0, 1, 2 ou 3, et
- avec la condition qu'au moins un motif siloxy (A2) a pour formule Si0_{4/2}.

4. Article adhésif sur la peau selon la revendication 3 dans lequel la résine silicone **A1**a pour formule MD^{Vi}Q dans laquelle:
- M est un motif siloxy monovalent de formule R₃SiO_{1/2}
- D^{Vi} est un motif siloxy divalent de formule RXSiO_{2/2}, et
- Q est un motif siloxy tétravalent de formule SiO_{4/2},
- avec le radical X correspondant à un groupement vinyle, et
- le radical R correspondant à un groupement hydrocarboné monovalent choisi parmi les groupes alkyles ayant de 1 à 8 atomes de carbone inclus tels que les groupes méthyle, éthyle, propyle et 3,3,3-trifluoropropyle, les groupes cycloalkyles comme les groupes cyclohexyle, cycloheptyle, cyclooctyle et les groupes aryle tels que xylyle, tolyle et phényle.

5. Article adhésif sur la peau selon la revendication 1 **caractérisé en ce que** le support **S** est un film souple continu en polyuréthane qui est perméable à l'air et imperméable aux fluides.

6. Article adhésif sur la peau selon l'une quelconque des revendications 1 et 5 **caractérisé en ce que** le support S qui est un film souple en polyuréthane comporte sur au moins une partie de la face de dessous **S2** un adhésif sensible à la pression.

7. Article adhésif sur la peau selon l'une quelconque des revendications 1, 5 et 6 **caractérisé en ce que** le support **S** est un film souple perforé en polyuréthane ou un film souple continu en polyuréthane ayant une face de dessus **S1** et une face de dessous **S2** et qui est imperméable à l'air et aux fluides dans ses parties comprises entre les perforations..

8. Article adhésif sur la peau selon la revendication 7 **caractérisé en ce que** les perforations du support **S** sont circulaires et ont un diamètre de 50 µm à 10 mm.

9. Article adhésif sur la peau selon la revendication 8 **caractérisé en ce qu'**il comprend une ou plusieurs couches **N** comprenant un corps absorbant **O,** éventuellement séparées par une ou plusieurs couches intermédiaires **P,** placée(s) sur le support **S** du côté de la face de dessous **S2** du support **S.**

10. Article adhésif sur la peau selon la revendication 9 dans lequel le corps absorbant **O** est choisi parmi le groupe constitué par : une mousse hydrophile, un tampon en tissu, un hydrogel, un hydrocolloïde et un alginate.

11. Article adhésif sur la peau selon la revendication 1 **caractérisé en ce que** :
- le support **S** est un film souple perforé en polyuréthane, ayant une face de dessus **S1** et une face de dessous **S2**,
- le primaire d'accrochage **C1** est sur la face de dessus **S1** dudit support **S** et a été appliqué de façon discontinue de manière à empêcher le bouchage des perforations du support **S**, et
- la couche **D1** constituée par un gel silicone **E** a été appliquée de façon discontinue de manière à empêcher le bouchage des perforations du support **S.**

12. Article adhésif sur la peau selon la revendication 1 **caractérisé en ce que** :
- le primaire d'accrochage **C1** est appliqué sur au moins une partie ou sur la totalité de la face de dessus **S1** et sur une partie ou sur la totalité de la face de dessous **S2** dudit support **S**, et
- au moins une couche **D1** constituée par un gel silicone **E** obtenu par réticulation d'une composition silicone **Y** réticulable par une réaction d'hydrosilylation a été appliquée sur ledit primaire d'accrochage **C1** présent sur la face de dessus **S1** et sur la face de dessous **S2** dudit support **S**.

13. Article adhésif sur la peau selon l'une quelconque des revendications précédentes **caractérisé en ce que** l'épaisseur du support **S** est comprise entre 5 et 200 µm, de préférence entre 10 et 75 µm.

14. Article adhésif sur la peau selon l'une quelconque des revendications précédentes **caractérisé en ce qu'**il s'agit d'un pansement à usage médical ou paramédical.

15. Procédé de préparation d'un article adhésif sur la peau tel que décrit selon l'une quelconque des revendications 1 à 14 **caractérisé en ce qu'**il comprend :
- une première étape consistant à déposer sur ledit support **S** des quantités suffisantes de la composition silicone **X** telle que décrite selon la revendication 1, 2, 3 ou 4, et à faire réticuler la composition silicone **X,** de préférence à une température comprise entre 60 et 200°C, pendant un temps suffisant de manière à former ledit primaire d'accrochage **C1,**
- une deuxième étape consistant à déposer sur ledit primaire d'accrochage **C1** des quantités suffisantes de la composition silicone **Y** telle que décrite selon la revendication 1 et à faire réticuler la composition silicone **Y,** de préférence à une température comprise entre 60 et 200°C, pendant un temps suffisant de manière à former la dite couche **D1.**

16. Procédé de préparation d'un article adhésif sur la peau tel que décrit selon l'une quelconque des revendications 1 à 14 **caractérisé en ce qu'**il comprend :
- une étape consistant à déposer de façon successive sur ledit support S des quantités suffisantes:
• de la composition silicone **X** telle que décrite selon la revendication 1, 2, 3 ou 4,
• puis ensuite de la composition silicone **Y** telle que décrite selon la revendication 1 sur la composition silicone **X,**
puis de faire réticuler lesdites compositions silicones **X** et **Y,** de préférence à une température comprise entre 60 et 200°C, pendant un temps suffisant de manière à former ledit primaire d'accrochage **C1** et la dite couche **D1.**

17. Procédé selon la revendication 16 **caractérisé en ce que** pour ladite composition silicone **X** les quantités en poids des organopolysiloxanes **A** et **B** sont déterminées de manière à ce que le rapport RHalk =1 avec RHalk = nH/tAlk et :
- nH = nombre de mole d'atome d'hydrogène directement lié à un atome de silicium de l'organopolysiloxane **B,** et
- tAlk = nombre de mole d'alcényle directement lié à un atome de silicium de l'organopolysiloxane **A.**

## Patentansprüche

1. Auf Haut haftender Artikel, umfassend:
• einen Träger S, der ein nachgiebiger Film aus Polyurethan mit einer oberen Fläche S1 und einer unteren Fläche S2 ist,
• mindestens eine primäre Befestigung C1, die auf mindestens einem Teil oder auf der Gesamtheit der oberen Fläche S1 des Trägers S aufgebracht ist,
• mindestens eine Schicht D1, welche aus einem Silikongel E besteht, das durch Vernetzung einer Silikon-Zusammensetzung Y erhalten wird, die durch eine Hydrosilylierungsreaktion vernetzbar ist, und welche auf die erste Befestigung C1 gegenüber dem Träger S aufgebracht wurde, und
• gegebenenfalls mindestens eine Schutzschicht F, die aus einem ablösbaren Schutzmaterial besteht und auf die Schicht D1 aufgebracht wurde,
• wobei die erste Befestigung C1 **dadurch gekennzeichnet ist, dass** diese aus einem Silikonelastomer besteht, das kein Silikongel ist, und das durch Vernetzung einer Silikon-Zusammensetzung X erhalten wird, bestehend aus:
1) mindestens einem Organopolysiloxan A, umfassend, pro Molekül, mindestens zwei Vinylreste, die jeweils mit einem Siliciumatom verbunden sind, und bestehend aus:
(i) mindestens zwei Siloxy-Einheiten mit der Formel (A1):
YₐZ_{b}SiO_{(4-(a+b)/2} (A1)
wobei:
- Y eine C₂ bis C₆-Alkenylgruppe und vorzugsweise eine Vinylgruppe darstellt,
- Z eine einwertige Kohlenwasserstoffgruppe darstellt, ausgewählt aus Alkylgruppen mit 1 bis einschließlich 8 Kohlenstoffatomen, wie Methyl-, Ethyl-, Propyl- und 3,3,3-Trifluorpropylgruppen, Cycloalkylgruppen, wie Cyclohexyl-, Cycloheptyl-, Cyclooctylgruppen, und Arylgruppen, wie Xylyl, Tolyl und Phenyl,
- a und b ganze Zahlen darstellen, wobei a 1, 2 oder 3 ist, b 0, 1 oder 2 ist, und (a+b) 1, 2 oder 3 ist;
(ii) und gegebenenfalls umfassend andere Siloxy-Einheiten mit der Formel (A2) :
Z_{c}SiO_{(4-c)/2} (A2)
wobei:
- Z dieselbe Bedeutung hat wie oben, und
- c eine ganze Zahl darstellt, die 0, 1, 2 oder 3 ist;
2) mindestens einem Organosiloxan B, umfassend, pro Molekül, mindestens drei Wasserstoffatome, die jeweils an ein Siliciumatom gebunden sind, und bestehend aus:
(i) mindestens drei Siloxy-Einheiten mit der Formel (B1):
HZ_{b}SiO_{(3-b)/2} (B1)
wobei:
- H ein Wasserstoffatom ist,
- Z eine einwertige Kohlenwasserstoffgruppe darstellt, ausgewählt aus Alkylgruppen mit 1 bis einschließlich 8 Kohlenstoffatomen, wie Methyl-, Ethyl-, Propyl- und 3,3,3-Trifluorpropylgruppen, Cycloalkylgruppen, wie Cyclohexyl-, Cycloheptyl-, Cyclooctylgruppen, und Arylgruppen, wie Xylyl, Tolyl und Phenyl,
- b 0, 1 oder 2 ist;
(ii) und gegebenenfalls umfassend andere Siloxy-Gruppen mit der Formel (B2):
Z_{c}SiO_{(4-c)/2} (B2)
wobei:
- Z dieselbe Bedeutung hat wie oben, und
- c eine ganze Zahl darstellt, die 0, 1, 2 oder 3 ist;
3) mindestens einem Hydrosilylierungskatalysator C,
4) mindestens einem Inhibitor D der Hydrosilylierungsreaktion,
5) gegebenenfalls mindestens einem Stabilisatoradditiv K, und
7) gegebenenfalls mindestens einem Silikonharz M mit Hydroxylgruppen und ohne Alkenyl- oder Hydrogenosilylgruppen SiH, und
- mit der Maßgabe, dass die Gewichtsmengen der Organopolysiloxane A und B derart bestimmt werden, dass das Verhältnis RHalk = nH/tAlk und 1,00 ≤ RHalk ≤ 4, 5, wobei:
- nH = Molzahl eines Wasserstoffatoms, das direkt an ein Siliciumatom des Organopolysiloxans B gebunden ist, und
- tAlk = Molzahl von Alkyl, das direkt an ein Siliciumatom des Organopolysiloxans A gebunden ist.

2. Auf Haut haftender Artikel nach Anspruch 1, **dadurch gekennzeichnet, dass** die Gewichtsmengen der Organopolysiloxane A und B derart bestimmt werden, dass 1,50 ≤ RHalk ≤ 4,0.

3. Auf Haut haftender Artikel nach Anspruch 1, wobei die Silikon-Zusammensetzung X mindestens zwei Organopolysiloxane A gemäß Anspruch 1 enthält, und **dadurch gekennzeichnet, dass** mindestens eine ein Silikonharz A1 ist, bestehend aus:
(i) mindestens zwei Siloxy-Einheiten mit der Formel (A1):
YₐZ_{b}SiO_{(4-(a+b)/2} (A1)
wobei:
- Y eine C₂-C₆-Alkenylgruppe und vorzugsweise eine Vinylgruppe darstellt,
- Z eine einwertige Kohlenwasserstoffgruppe darstellt, ausgewählt aus Alkylgruppen mit 1 bis einschließlich 8 Kohlenstoffatomen, wie Methyl-, Ethyl-, Propyl- und 3,3,3-Trifluorpropylgruppen, Cycloalkylgruppen, wie Cyclohexyl-, Cycloheptyl-, Cyclooctylgruppen, und Arylgruppen, wie Xylyl, Tolyl und Phenyl,
- a und b ganze Zahlen darstellen, wobei a 1, 2 oder 3 ist, b 0, 1 oder 2 ist, und (a+b) 1, 2 oder 3 ist; und vorzugsweise a = 1;
(ii) und anderen Siloxy-Einheiten mit der Formel (A2) :
Z_{c}SiO_{(4-c)/2} (A2)
wobei:
- Z dieselbe Bedeutung hat wie oben,
- c eine ganze Zahl darstellt, die 0, 1, 2 oder 3 ist, und
- mit der Maßgabe, dass mindestens eine Siloxy-Einheit (A2) die Formel SiO_{4/2} aufweist.

4. Auf Haut haftender Artikel nach Anspruch 3, wobei das Silikonharz A1 die Formel MD^{Vi}Q aufweist, wobei:
- M eine einwertige Siloxy-Einheit mit der Formel R₃SiO_{1/2} ist,
- D^{Vi} eine zweiwertige Siloxy-Einheit mit der Formel RXSiO_{2/2} ist, und
- Q eine vierwertige Siloxy-Einheit mit der Formel SiO_{4/2} ist,
- wobei der Rest X einer Vinylgruppe entspricht, und
- der Rest R einer einwertigen Kohlenwasserstoffgruppe entspricht, ausgewählt aus Alkylgruppen mit 1 bis einschließlich 8 Kohlenstoffatomen, wie Methyl-, Ethyl-, Propyl- und 3,3,3-Trifluorpropylgruppen, Cycloalkylgruppen, wie Cyclohexyl-, Cycloheptyl-, Cyclooctylgruppen, und Arylgruppen, wie Xylyl, Tolyl und Phenyl.

5. Auf Haut haftender Artikel nach Anspruch 1, **dadurch gekennzeichnet, dass** der Träger S ein kontinuierlicher nachgiebiger Film aus Polyurethan ist, der für Luft permeabel ist und für Fluids impermeabel ist.

6. Auf Haut haftender Artikel nach einem der Ansprüche 1 und 5, **dadurch gekennzeichnet, dass** der Träger S, der ein nachgiebiger Film aus Polyurethan ist, auf mindestens einem Teil der unteren Fläche S2 ein druckempfindliches Haftmittel umfasst.

7. Auf Haut haftender Artikel nach einem der Ansprüche 1, 5 und 6, **dadurch gekennzeichnet, dass** der Träger S ein perforierter nachgiebiger Film aus Polyurethan oder ein kontinuierlicher nachgiebiger Film aus Polyurethan mit einer oberen Fläche S1 und einer unteren Fläche S2 ist, und der für Luft und Fluids in seinen Teilen, die zwischen den Perforationen liegen, impermeabel ist.

8. Auf Haut haftender Artikel nach Anspruch 7, **dadurch gekennzeichnet, dass** die Perforationen des Trägers S kreisförmig sind und einen Durchmesser von 50 µm bis 10 mm aufweisen.

9. Auf Haut haftender Artikel nach Anspruch 8, **dadurch gekennzeichnet, dass** dieser eine oder mehrere Schichten N umfasst, umfassend einen absorbierenden Körper O, gegebenenfalls getrennt durch eine oder mehrere Zwischenschichten P, die auf dem Träger S auf der Seite der unteren Fläche S2 des Trägers S platziert sind.

10. Auf Haut haftender Artikel nach Anspruch 9, wobei der absorbierende Körper O ausgewählt ist aus der Gruppe bestehend aus: einem hydrophilen Schaum, einem Gewebekissen, einem Hydrogel, einem Hydrokolloid und einem Alginat.

11. Auf Haut haftender Artikel nach Anspruch 1, **dadurch gekennzeichnet, dass**:
- der Träger S ein perforierter nachgiebiger Film aus Polyurethan mit einer oberen Fläche S1 und einer unteren Fläche S2 ist,
- die primäre Befestigung C1 auf der oberen Fläche S1 des Trägers S ist und auf diskontinuierliche Weise aufgebracht wurde, um das Verstopfen der Perforationen des Trägers S zu verhindern, und
- die Schicht D1, die aus einem Silikongel E besteht, auf diskontinuierliche Weise aufgebracht wurde, um das Verstopfen der Perforationen des Trägers S zu verhindern.

12. Auf Haut haftender Artikel nach Anspruch 1, **dadurch gekennzeichnet, dass**:
- die primäre Befestigung C1 auf mindestens einem Teil oder auf der Gesamtheit der oberen Fläche S1 und auf einem Teil oder auf der Gesamtheit der unteren Fläche S2 des Trägers S aufgebracht ist, und
- mindestens eine Schicht D1, welche aus einem Silikongel E besteht, das durch Vernetzung einer Silikon-Zusammensetzung Y erhalten wird, die durch eine Hydrosilylierungsreaktion vernetzbar ist, auf die erste Befestigung C1, die auf der oberen Fläche S1 vorliegt, und auf der unteren Fläche S2 des Trägers S aufgebracht wurde.

13. Auf Haut haftender Artikel nach einem der vorhergehenden Ansprüche, **dadurch gekennzeichnet, dass** die Dicke des Trägers S zwischen 5 und 200 µm, vorzugsweise zwischen 10 und 75 µm, beträgt.

14. Auf Haut haftender Artikel nach einem der vorhergehenden Ansprüche, **dadurch gekennzeichnet, dass** es sich um einen Wundverband zur medizinischen oder paramedizinischen Verwendung handelt.

15. Verfahren zur Herstellung eines auf Haut haftenden Artikels, wie nach einem der Ansprüche 1 bis 14 beschrieben, **dadurch gekennzeichnet, dass** dieses umfasst:
- einen ersten Schritt, bestehend aus dem Abscheiden, auf dem Träger S, ausreichender Mengen der Silikon-Zusammensetzung X, wie nach Anspruch 1, 2, 3 oder 4 beschrieben, und Vernetzen der Silikon-Zusammensetzung X, vorzugsweise bei einer Temperatur zwischen 60 und 200 °C, während einer ausreichenden Zeit, um die primäre Befestigung C1 zu bilden,
- einen zweiten Schritt, bestehend aus dem Abscheiden, auf der primären Befestigung C1, ausreichender Mengen der Silikon-Zusammensetzung Y, wie nach Anspruch 1 beschrieben, und Vernetzen der Silikon-Zusammensetzung Y, vorzugsweise bei einer Temperatur zwischen 60 und 200 °C, während einer ausreichenden Zeit, um die Schicht D1 zu bilden.

16. Verfahren zur Herstellung eines auf Haut haftenden Artikels, wie nach einem der Ansprüche 1 bis 14 beschrieben, **dadurch gekennzeichnet, dass** dieses umfasst:
- einen Schritt, bestehend aus dem Abscheiden auf aufeinanderfolgende Weise, auf dem Träger S, ausreichender Mengen:
• der Silikon-Zusammensetzung X, wie nach einem der Ansprüche 1, 2, 3 oder 4 beschrieben,
• dann anschließend der Silikon-Zusammensetzung Y, wie nach Anspruch 1 beschrieben, auf der Silikon-Zusammensetzung X,
dann Vernetzen der Silikon-Zusammensetzungen X und Y, vorzugsweise bei einer Temperatur zwischen 60 und 200 °C, während einer ausreichenden Zeit, um die primäre Befestigung C1 und die Schicht D1 zu bilden.

17. Verfahren nach Anspruch 16, **dadurch gekennzeichnet, dass** für die Silikon-Zusammensetzung X die Gewichtsmengen der Organopolysiloxane A und B derart bestimmt werden, dass das Verhältnis RHalk = 1, wobei RHalk = nH/tAlk und:
- nH = Molzahl eines Wasserstoffatoms, das direkt an ein Siliciumatom des Organopolysiloxans B gebunden ist, und
- tAlk = Molzahl von Alkyl, das direkt an ein Siliciumatom des Organopolysiloxans A gebunden ist.

## Claims

1. Skin-adhesive item comprising:
• a support **S** which is a flexible polyurethane film having a top face **S1** and a bottom face **S2**,
• at least one tie primer **C1** applied to at least one part or to all of the top face **S1** of said support **S**,
• at least one layer **D1** made up of a silicone gel **E** obtained by crosslinking a crosslinkable silicone composition **Y** by means of a hydrosilylation reaction, which has been applied to said tie primer **C1** facing said support **S**, and
• optionally at least one protective layer **F** consisting of a peel-off protective material and applied to said layer **D1,**
• said tie primer **C1** being **characterized in that** it consists of a silicone elastomer which is not a silicone gel and which is obtained by crosslinking a silicone composition **X** consisting of:
1) at least one organopolysiloxane **A** comprising, per molecule, at least two vinyl radicals each bonded to a silicon atom and consisting:
(i) of at least two siloxy units of formula (A1):
YₐZ_{b}SiO_{(4-(a+b)/2} (A1)
wherein:
- Y represents a C₂ to C₆ alkenyl group, and preferably a vinyl group,
- Z represents a monovalent hydrocarbon-based group chosen from alkyl groups having from 1 to 8 carbon atoms inclusive, such as methyl, ethyl, propyl and 3,3,3-trifluoropropyl groups, cycloalkyl groups, such as cyclohexyl, cycloheptyl and cyclooctyl groups, and aryl groups such as xylyl, tolyl and phenyl groups,
- a and b represent integers, a being 1, 2 or 3, b being 0, 1 or 2 and (a+b) being 1, 2 or 3;
(ii) and optionally comprising other siloxy units of formula (A2):
Z_{c}SiO_{(4-c)/2} (A2)
wherein:
- Z has the same meaning as above, and
- c represents an integer which is 0, 1, 2 or 3;
2) at least one organopolysiloxane **B** comprising, per molecule, at least three hydrogen atoms each bonded to a silicon atom and consisting:
(i) of at least three siloxy units of formula (B1) :
HZ_{b}SiO_{(3-b)/2} (B1)
wherein:
- H is a hydrogen atom,
- Z represents a monovalent hydrocarbon-based group chosen from alkyl groups having from 1 to 8 carbon atoms inclusive, such as methyl, ethyl, propyl and 3,3,3-trifluoropropyl groups, cycloalkyl groups, such as cyclohexyl, cycloheptyl and cyclooctyl groups, and aryl groups such as xylyl, tolyl and phenyl groups,
- b is 0, 1 or 2;
(ii) and optionally comprising other siloxy units of formula (B2):
Z_{c}SiO_{(4-c)/2} (B2)
wherein:
- Z has the same meaning as above, and
- c represents an integer which is 0, 1, 2, or 3;
3) at least one hydrosilylation catalyst **C**;
4) at least one hydrosilylation reaction inhibitor **D**;
5) optionally at least one stabilizing additive **K**; and
7) optionally at least one silicone resin **M** which has hydroxyl groups and is free of alkenyl or hydrosilyl SiH groups, and
- with the proviso that the amounts by weight of the organopolysiloxanes **A** and **B** are determined such that the RHalk = nH/tAlk ratio is 1.00 ≤ RHalk ≤ 4.5 with:
- nH = number of moles of hydrogen atom directly bonded to a silicon atom of the organopolysiloxane **B**, and
- tAlk = number of moles of alkenyl directly bonded to a silicon atom of the organopolysiloxane **A**.

2. Skin-adhesive item according to Claim 1, **characterized in that** the amounts by weight of the organopolysiloxanes **A** and **B** are determined such that 1.50 ≤ RHalk ≤ 4.0.

3. Skin-adhesive item according to Claim 1, wherein the silicone composition **X** contains at least two organopolysiloxanes **A** according to Claim 1, and **characterized in that** at least one is a silicone resin **A1** consisting:
(i) of at least two siloxy units of formula (A1) :
YₐZ_{b}SiO_{(4-(a+b)/2} (A1)
wherein:
- Y represents a C₂ to C₆ alkenyl group, and preferably a vinyl group,
- Z represents a monovalent hydrocarbon-based group chosen from alkyl groups having from 1 to 8 carbon atoms inclusive, such as methyl, ethyl, propyl and 3,3,3-trifluoropropyl groups, cycloalkyl groups, such as cyclohexyl, cycloheptyl and cyclooctyl groups, and aryl groups such as xylyl, tolyl and phenyl groups,
- a and b represent integers, a being 1, 2 or 3, b being 0, 1 or 2 and (a+b) being 1, 2 or 3; and preferably a = 1;
(ii) and other siloxy units of formula (A2):
Z_{c}SiO_{(4-c)/2} (A2)
wherein:
- Z has the same meaning as above,
- c represents an integer which is 0, 1, 2 or 3, and
- with the proviso that at least one siloxy unit (A2) has the formula SiO_{4/2}.

4. Skin-adhesive item according to Claim 3, wherein the silicone resin **A1** has the formula MD^{Vi}Q wherein:
- M is a monovalent siloxyl unit of formula R₃SiO_{1/2}
- D^{Vi} is a divalent siloxyl unit of formula RXSiO_{2/2}, and
- Q is a tetravalent siloxyl unit of formula SiO_{4/2},
- with the radical X corresponding to a vinyl group, and
- the radical R corresponding to a monovalent hydrocarbon-based group chosen from alkyl groups having from 1 to 8 carbon atoms inclusive, such as methyl, ethyl, propyl and 3,3,3-trifluoropropyl groups, cycloalkyl groups, such as cyclohexyl, cycloheptyl and cyclooctyl groups, and aryl groups such as xylyl, tolyl and phenyl groups.

5. Skin-adhesive item according to Claim 1, **characterized in that** the support **S** is a continuous flexible polyurethane film which is permeable to air and impermeable to fluids.

6. Skin-adhesive item according to any one of Claims 1 to 5, **characterized in that** the support **S,** which is a flexible polyurethane film, comprises, on at least one part of the bottom face **S2,** a pressure-sensitive adhesive.

7. Skin-adhesive item according to any one of Claims 1, 5 and 6, **characterized in that** the support **S** is a perforated flexible polyurethane film or a continuous flexible polyurethane film which has a top face **S1** and a bottom face **S2** and which is impermeable to air and fluids in its parts between the perforations.

8. Skin-adhesive item according to Claim 7, **characterized in that** the perforations of the support **S** are circular and have a diameter of from 50 µm to 10 mm.

9. Skin-adhesive item according to Claim 8, **characterized in that** it comprises one or more layers **N** comprising an absorbent body **O**, optionally separated by one or more intermediate layers **P**, placed on the support **S** on the side of the bottom face **S2** of the support **S.**

10. Skin-adhesive item according to Claim 9, wherein the absorbent body **O** is chosen from the group made up of: a hydrophilic foam, a fabric pad, a hydrogel, a hydrocolloid and an alginate.

11. Skin-adhesive item according to Claim 1, **characterized in that**:
- the support **S** is a perforated flexible polyurethane film, having a top face **S1** and a bottom face **S2,**
- the tie primer **C1** is on the top face **S1** of said support **S** and has been applied discontinuously so as to prevent blocking of the perforations of the support **S**, and
- the layer **D1** made up of a silicone gel **E** has been applied discontinuously so as to prevent blocking of the perforations of the support **S**.

12. Skin-adhesive item according to Claim 1, **characterized in that**:
- the tie primer **C1** is applied on at least one part or on all of the top face **S1** and on a part or on all of the bottom face **S2** of said support **S**, and
- at least one layer **D1** made up of a silicone gel **E** obtained by crosslinking a crosslinkable silicone composition **Y** by means of a hydrosilylation reaction has been applied on said tie primer **C1** present on the top face **S1** and on the bottom face **S2** of said support **S.**

13. Skin-adhesive item according to any one of the preceding claims, **characterized in that** the thickness of the support **S** is between 5 and 200 pm, preferably between 10 and 75 pm.

14. Skin-adhesive item according to any one of the preceding claims, **characterized in that** it is a dressing for medical or paramedical use.

15. Process for preparing a skin-adhesive item as described in any one of Claims 1 to 14, **characterized in that** it comprises:
- a first step consisting in depositing, on said support **S**, sufficient amounts of the silicone composition **X** as described in Claim 1, 2, 3 or 4, and in crosslinking the silicone composition **X**, preferably at a temperature of between 60 and 200°C, for a sufficient time so as to form said tie primer **C1**,
- a second step consisting in depositing, on said tie primer **C1**, sufficient amounts of the silicone composition **Y** as described in Claim 1 and in crosslinking the silicone composition **Y**, preferably at a temperature of between 60 and 200°C, for a sufficient time so as to form said layer **D1.**

16. Process for preparing a skin-adhesive item as described in any one of Claims 1 to 14, **characterized in that** it comprises:
- a step consisting in successively depositing, on said support **S,** sufficient amounts:
• of the silicone composition **X** as described in Claim 1, 2, 3 or 4,
• then subsequently of the silicone composition **Y** as described in Claim 1 on the silicone composition **X**,
then crosslinking said silicone compositions **X** and **Y**, preferably at a temperature of between 60 and 200°C, for a sufficient time so as to form said tie primer **C1** and said layer **D1.**

17. Process according to Claim 16, **characterized in that**, for said silicone composition **X**, the amounts by weight of the organopolysiloxanes **A** and **B** are determined such that the ratio RHalk = 1 with RHalk = nH/tAlk and:
- nH = number of moles of hydrogen atom directly bonded to a silicon atom of the organopolysiloxane **B**, and
- tAlk = number of moles of alkenyl directly bonded to a silicon atom of the organopolysiloxane **A.**
